# EUROPÄISCHE PATENTSCHRIFT

(11) **EP 0 808 310 B1**
(45) Veröffentlichungstag und Bekanntmachung des Hinweises auf die Patenterteilung: **02.01.2002**
(21) Anmeldenummer: 96901767.2
(22) Anmeldetag: 26.01.1996
(51) Int. Cl.: C07D 239/54, C07C 237/18

(54) **3-(4-CYANOPHENYL)URACILE**
3-(4-CYANOPHENYL)URACILS
3-(4-CYANOPHENYLE)URACILES

(30) Priorität: 09.02.1995 DE 19504188
(43) Veröffentlichungstag der Anmeldung: 26.11.1997
(73) Patentinhaber: BASF AKTIENGESELLSCHAFT, 67056 Ludwigshafen (DE)
(72) Erfinder: KLINTZ, Ralf, D-67269 Gruenstadt (DE); SCHÄFER, Peter, D-67308 Ottersheim (DE); HAMPRECHT, Gerhard, D-69469 Weinheim (DE); HEISTRACHER, Elisabeth, D-67061 Ludwigshafen (DE); VON DEM BUSSCHE-HÜNNEFELD, Christoph-Sweder, D-68199 Mannheim (DE); GÖTZ, Norbert, D-67547 Worms (DE); HARREUS, Albrecht, D-67063 Ludwigshafen (DE); WESTPHALEN, Karl-Otto, D-67346 Speyer (DE); WALTER, Helmut, D-67283 Obrigheim (DE); MISSLITZ, Ulf, D-67433 Neustadt (DE)
(86) Internationale Anmeldenummer: EP9600312
(87) Internationale Veröffentlichungsnummer: WO9624590

(56) Entgegenhaltungen:
- EP-A- 0 255 047
- EP-A- 0 260 621
- EP-A- 0 542 685

## Beschreibung

Die vorliegende Erfindung betrifft 3-(4-Cyanophenyl)uracile der allgemeinen Formel I 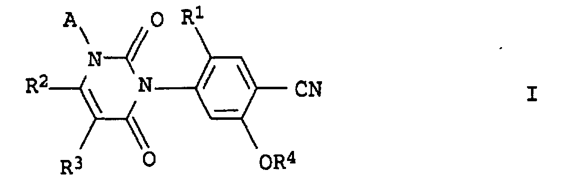 in der die Variablen folgende Bedeutung haben:
- A: Methyl oder Amino;
- R¹: Wasserstoff oder Fluor;
- R²: C₁-C₄-Halogenalkyl;
- R³: Wasserstoff oder Halogen;
- R⁴: Wasserstoff, C₁-C₄-Halogenalkyl,
C₁-C₄-Alkyl, C₃-C₆-Cycloalkyl, C₃-C₄-Alkenyl, C₃-C₄-Alkinyl, (C₁-C₄-Alkyl)carbonyl, C₁-C₄-Cyanoalkyl, C₁-C₄-Alkoxy-C₁-C₄-alkyl, CH₂-CO-XR⁵ oder CH(CH₃)-CO-XR⁵;
- X: eine chemische Bindung, Sauerstoff oder -N(R⁶)-;
- R⁵: Wasserstoff, C₁-C₆-Alkyl, C₃-C₈-Cycloalkyl, C₃-C₆-Alkenyl, C₃-C₆-Alkinyl, C₁-C₆-Alkoxy-C₁-C₆-alkyl oder (C₁-C₆-Alkoxy)carbonyl-C₁-C₆-alkyl;
- R⁶: Wasserstoff, C₁-C₆-Alkyl oder C₁-C₆-Alkoxy.

Außerdem betrifft die Erfindung
- die Verwendung der Verbindungen I als Herbizide und/oder zur Desikkation und/oder Defoliation von Pflanzen,
- herbizide Mittel und Mittel zur Desikkation und/oder Defoliation von Pflanzen, welche die Verbindungen I als wirksame Substanzen enthalten,
- Verfahren zur Bekämpfung von unerwünschtem Pflanzenwuchs und zur Desikkation und/oder Defoliation von Pflanzen mit den Verbindungen I,
- Verfahren zur Herstellung der Verbindungen I und von herbiziden Mitteln und Mitteln zur Desikkation und/oder Defoliation von Pflanzen unter Verwendung der Verbindungen I, sowie
- Zwischenprodukte der Formeln III und IV, aus denen die Verbindungen I erhältlich sind.

Bezüglich der Verbindungen I mit A = Methyl ist die EP-A 255 047 von besonderer Bedeutung, denn in dieser Druckschrift werden bereits ganz allgemein 3-Aryluracile der Formel II 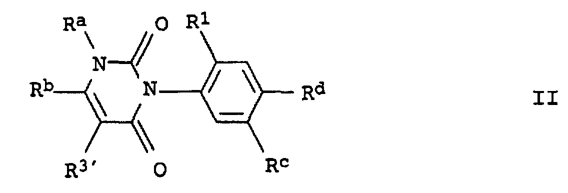 wobei
R^{a} für Wasserstoff, C₁₋₄-Alkyl, C₁₋₄-Halogenalkyl, Formyl oder C₂₋₆-Alkanoyl,
R^{b} für C₁₋₄-Alkyl oder C₁₋₄-Halogenalkyl,
R³' für Wasserstoff, Halogen oder C₁-C₄-Alkyl,
R^{c} für eine Ethergruppe oder einen Rest R-CO-O-, R-CS-O- oder R-SO₂-O- und
R^{d} für Halogen oder Cyano stehen, sowie die Salze der Verbindungen II mit R^{a} = Wasserstoff, als Herbizide beschrieben.

Beispiele für 3-Phenyluracile, bei denen der Phenylring eine Cyanogruppe in para-Stellung zum Uracil-Rest (R^{d}) trägt, sowie deren herbizide Wirkung sind dieser Schrift jedoch nicht zu entnehmen.

Bestimmte 1-Amino-3-phenyluracile, die jedoch keine Cyanogruppe am Phenylring tragen, werden bereits in der EP-A 517 181 und der JP-A 05/025 143 als Herbizide gelehrt.

EP-A-260 621 und EP-A-542 685 beschreiben speziell substituierte 3-(4-Cyanophenyl)uracile sowie deren Verwendung zur Unkrautvernichtung.

Die herbiziden oder desikkant/defolianten Eigenschaften der bekannten Verbindungen sind jedoch nicht immer voll befriedigend. Es lagen daher dieser Erfindung neue, insbesondere herbizid wirksame Verbindungen als Aufgabe zugrunde, mit denen sich unerwünschte Pflanzen besser als bisher gezielt bekämpfen lassen.

Die Aufgabe erstreckt sich auch auf die Bereitstellung neuer desikkant/defoliant wirksamer Verbindungen.

Demgemäß wurden die 3-(4-Cyanophenyl)uracile der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Des weiteren wurde gefunden, daß die Verbindungen I auch zur Defoliation und Desikkation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und/oder Defoliation von Pflanzen, Verfahren zur Herstellung dieser Mittel und Verfahren zur Desikkation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die für die Substituenten R¹ bis R⁶ oder als Reste an Phenylringen- oder Heterocyclen genannten organischen Molekülteile stellen - wie die Bedeutung Halogen - Sammelbegriffe für individuelle Aufzählungen der einzelnen Gruppenmitglieder dar. Sämtliche Kohlenstoffketten, also alle Alkyl-, Halogenalkyl-, Alkoxy-, Alkylcarbonyl-, Alkenyl- und Alkinyl-Teile können geradkettig oder verzweigt sein. Sofern nicht anders angegeben tragen halogenierte Substituenten vorzugsweise ein bis fünf gleiche oder verschiedene Halogenatome.

Im einzelnen stehen beispielsweise:
- Halogen für: Fluor, Chlor, Brom oder Iod;
- C₁-C₄-Alkyl sowie die Alkyl-Teile von C₁-C₆-Alkoxy-C₁-C₆-alkyl und (C₁-C₆-Alkoxy)carbonyl-C₁-C₆-alkyl für:
   Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, 1,1-Dimethylpropyl, 1,2-Dimethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropl, 1,2,2-Trimethylpropyl, 1-Ethyl-1-methylpropyl oder 1-Ethyl-2-methylpropyl;
- C₁-C₄-Halogenalkyl für: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Petafluorethyl, 2-Fluorpropyl,3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₃-C₆-Alkenyl für: Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methyl-prop-2-en-1-yl oder 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-l-yl, 4-Methyl-pent-1-en-1-yl, l-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-put-3-en-1-yl, 1,2-Dimethyl-but-1-en-1-yl, 1,2-Dimetyl-put-2-en-1-yl, 1,2-Dimethyl-but-3-en-1-yl, 1,3-Dimetyl-put-1-en-1-yl, 1,3-Dimethyl-but-2-en-1-yl, 1,3-Dimetyl-put-3-en-1-yl, 2,2-Dimethyl-but-3-en-1-yl, 2,3-Dimetyl-put-1-en-1-yl, 2,3-Dimethyl-but-2-en-1-yl, 2,3-Dimetyl-put-3-en-1-yl, 3,3-Dimethyl-but-1-en-1-yl, 3,3-Dimetyl-put-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- C₃-C₆-Alkinyl sowie die Alkinyl-Teile von C₃-C₆-Alkinyloxy und (C₃-C₆-Alkinyl)carbonyl für: Prop-1-in-1-yl, Prop-2-in-1-yl, But-1-in-1-yl, But-1-in-3-yl, But-1-in-4-yl, But-2-in-1-yl, Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl;
- C₁-C₆-Alkoxy sowie die Alkoxy-Teile von C₁-C₆-Alkoxy-C₁-C₆-alkyl und (C₁-C₆-Alkoxy)carbonyl-C₁-C₆-alkyl für: Methoxy, Ethoxy, n-Propoxy, 1-Methylethoxy, n-Butoxy, 1-Methylpropoxy, 2-Methylpropoxy oder 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
- (C₁-C₄-Alkyl)carbonyl für: Methylcarbonyl, Ethylcarbonyl, n-Propylcarbonyl, 1-Methylethylcarbonyl, n-Butylcarbonyl, 1-Methylpropylcarbonyl, 2-Methylpropylcarbonyl, 1,1-Dimethylethylcarbonyl;
- C₃-C₈-Cycloalkyl für: Cyclopropyl, Cyclobutyl, Cyclopentyl, Cyclohexyl, Cycloheptyl und Cyclooctyl;

Im Hinblick auf die Verwendung der erfindungsgemäßen Verbindungen der Formel I als Herbizide und/oder als defoliant/desikkant wirksame Verbindungen haben die Variablen vorzugsweise folgende Bedeutungen, und zwar jeweils für sich allein oder in Kombination:
- A: Amino oder Methyl;
- R¹: Wasserstoff oder Fluor;
- R²: C₁-C₄-Haiogenalkyl, besonders bevorzugt Trifluormethyl, Chlordifluormethyl oder Pentafluorethyl;
- R³: Wasserstoff oder Halogen, insbesondere Wasserstoff, Chlor oder Brom;
- R⁴: Wasserstoff, C₁-C₄-Halogenalkyl, C₁-C₄-Alkyl, C₃-C₆-Cycloalkyl, C₃-C₄-Alkenyl, C₃-C₄-Alkinyl, (C₁-C₄-Alkyl)carbonyl, C₁-C₄-Alkoxy-C₁-C₄-alkyl, -CH₂-CO-XR⁵, -CH(CH₃)-CO-XR⁵ oder C₁-C₄-Cyanoalkyl wie Cyanomethyl, 1-Cyanoeth-1-yl, 2-Cyanoeth-1-yl, 1-Cyanoprop-1-yl, 2-Cyanoprop-1-yl, 3-Cyanoprop-1-yl, 1-Cyanoprop-2-yl, 2-Cyanoprop-2-yl, 1-Cyanobut-1-yl, 2-cyanobut-1-yl, 3-Cyanobut-1-yl, 4-Cyanobut-1-yl, 1-Cyanobut-2-yl, 2-Cyano-but-2-yl, 1-Cyanobut-3-yl, 2-Cyanobut-3-yl, 1-Cyano-2-methyl-prop-3-yl, 2-Cyano-2-methyl-prop-3-yl, 3-Cyano-2-methyl-prop-3-yl und 2-Cyano-methyl-prop-2-yl;
- X: eine chemische Bindung, Sauerstoff oder -N(R⁶)-;
- R⁵: Wasserstoff, C₁-C₆-Alkyl, C₃-C₈-Cycloalkyl, C₃-C₆-Alkenyl, C₃-C₆-Alkinyl, C₁-C₆-Alkoxy-C₁-C₆-alkyl oder (C₁-C₆-Alkoxy)carbonyl-C₁-C₆-alkyl, insbesondere Wasserstoff, C₁-C₆-Alkyl, C₃-C₈-Cycloalkyl, C₁-C₆-Alkoxy-C₁-C₆-alkyl oder (C₁-C₆-Alkoxy)carbonyl-C₁-C₆-alkyl,
- R⁶: Wasserstoff, C₁-C₆-Alkyl oder C₁-C₆-Alkoxy.

Ganz besonders bevorzugt sind die in der folgenden Tabelle 1 aufgeführten Verbindungen Ia (=̂ I mit A = Amino, R¹ = Fluor, R² = Trifluormethyl, R³ = Wasserstoff):

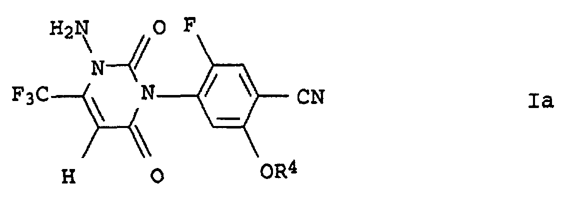

**Tabelle 1**

| Nr. | R⁴ |
|---|---|
| Ia.01 | H |
| Ia.02 | CH₃ |
| Ia.03 | C₂H₅ |
| Ia.04 | n-C₃H₇ |
| Ia.05 | CH(CH₃)₂ |
| Ia.06 | n-C₄H₉ |
| Ia.07 | i-C₄H₉ |
| Ia.08 | s-C₄H₉ |
| Ia.09 | C(CH₃)₃ |
| Ia.10 | Cyclopropyl |
| Ia.11 | Cyclobutyl |
| Ia.12 | Cyclopentyl |
| Ia.13 | Cyclohexyl |
| Ia.14 | CH₂CN |
| Ia.15 | CH₂CH₂CN |
| Ia.16 | CH(CH₃)CN |
| Ia.17 | C(CH₃)₂CN |
| Ia.18 | C(CH₃)₂CH₂CN |
| Ia.19 | CH₂Cl |
| Ia.20 | CH₂CH₂Cl |
| Ia.21 | CH(CH₃)CH₂Cl |
| Ia.22 | CH₂CF₃ |
| Ia.23 | CHCl₂ |
| Ia.24 | CF₂Cl |
| Ia.25 | CF₃ |
| Ia.26 | C₂F₅ |
| Ia.27 | CF₂H |
| Ia.28 | CH₂-CH=CH₂ |
| Ia.29 | CH(CH₃)-CH=CH₂ |
| Ia.30 | CH₂-CH=CH-CH₃ |
| Ia.31 | CH₂-C≡CH |
| Ia.32 | CH(CH₃)-C≡CH |
| Ia.33 | CH₂-COOH |
| Ia.34 | CH₂-CO-OCH₃ |
| Ia.35 | CH₂-CO-OC₂H₅ |
| Ia.36 | CH₂-CO-O-n-C₃H₇ |
| Ia.37 | CH₂-CO-OCH (CH₃)₂ |
| Ia.38 | CH(CH₃)-CO-OCH₃ |
| Ia.39 | CH(CH₃)-CO-OC₂H₅ |
| Ia.40 | CH(CH₃)-CO-O-n-C₃H₇ |
| Ia.41 | CH(CH₃)-CO-OC(CH₃)₂ |
| Ia.42 | CH₂-COO-(CH₂)₂-OCH₃ |
| Ia.43 | CH₂-COO-(CH₂)₂-OCH₃ |
| Ia.44 | CH(CH₃)-COO-(CH₂)₂-OCH₃ |
| Ia.45 | CH (CH₃)-COO-(CH₂)₂-OC₂H₅ |
| Ia.46 | CH₂-CONH₂ |
| Ia.47 | CH₂-CONHCH₃ |
| Ia.48 | CH₂-CONHC₂H₅ |
| Ia.49 | CH₂-CON(CH₃)₂ |
| Ia.50 | CH(CH₃)-CONH₂ |
| Ia.51 | CH(CH₃)-CONHCH₃ |
| Ia.52 | CH(CH₃)-CONHC₂H₅ |
| Ia.53 | CH(CH₃)-CON(CH₃)₂ |
| Ia.54 | CO-CH₃ |
| Ia.55 | CO-C₂H₅ |
| Ia.56 | CO-CH(CH₃)₂ |
| Ia.57 | CO-n-C₄H₉ |
| Ia.58 | CH₂-OCH₃ |
| Ia.59 | CH(CH₃)-OCH₃ |
| Ia.60 | CH(CH₃)-OC₂H₅ |
| Ia.61 | CH (CH₃)CH₂-OCH₃ |
| Ia.62 | CH₂-OC₂H₅ |

Des weiteren sind die folgenden 3-(4-Cyanophenyl)uracile der Formel I besonders bevorzugt:
- die Verbindungen Ib.01 - Ib.62, die sich von den entsprechenden Verbindungen Ia.01 - Ia.62 lediglich dadurch unterscheiden, daß R¹ Wasserstoff bedeutet: 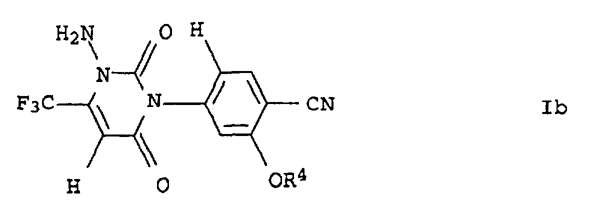
- die Verbindungen Ic.01 - Ic.62, die sich von den entsprechenden Verbindungen Ia.01 - Ia.62 lediglich dadurch unterscheiden, daß A Methyl bedeutet: 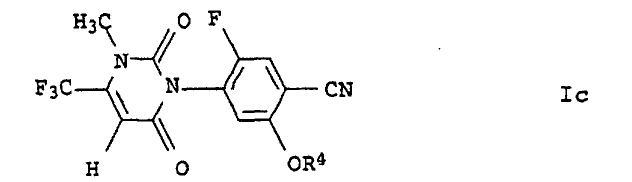
- die Verbindungen Id.01 - Id.62, die sich von den entsprechenden Verbindungen Ia.01 - Ia.62 lediglich dadurch unterscheiden, daß R¹ Wasserstoff und A Methyl bedeuten: 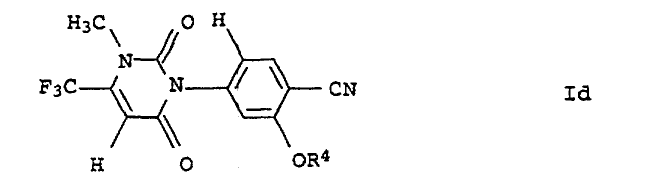
- die Verbindungen Ie.01 - Ie.62, die sich von den entsprechenden Verbindungen Ia.01 - Ia.62 lediglich dadurch unterscheiden, daß R³ Chlor bedeutet: 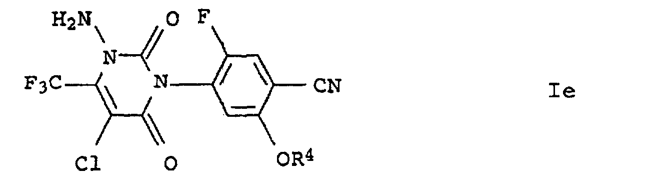
- die Verbindungen If.01 - If.62, die sich von den entsprechenden Verbindungen Ia.01 - Ia.62 lediglich dadurch unterscheiden, daß R³ Chlor und A Methyl bedeuten: 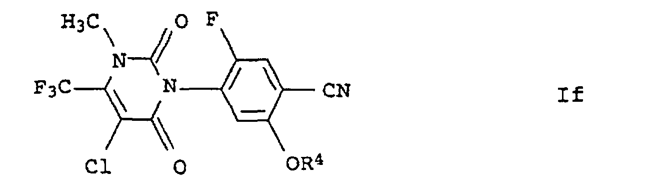
- die Verbindungen Ig.01 - Ig.62, die sich von den entsprechenden Verbindungen Ia.01 - Ia.62 lediglich dadurch unterscheiden, daß A Methyl, R¹ Wasserstoff und R³ Chlor bedeuten:

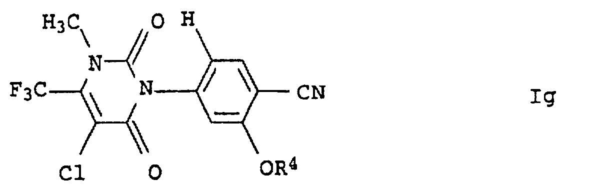

Die 3-(4-Cyanophenyl)uracile der Formel I sind auf verschiledene Weise erhältlich, beispielsweise nach einem der folgenden Verfahren:

### Verfahren A) :

Cyclisierung eines Enaminesters der Formel III oder eines Enamincarboxylats der Formel IV in Gegenwart einer Base:

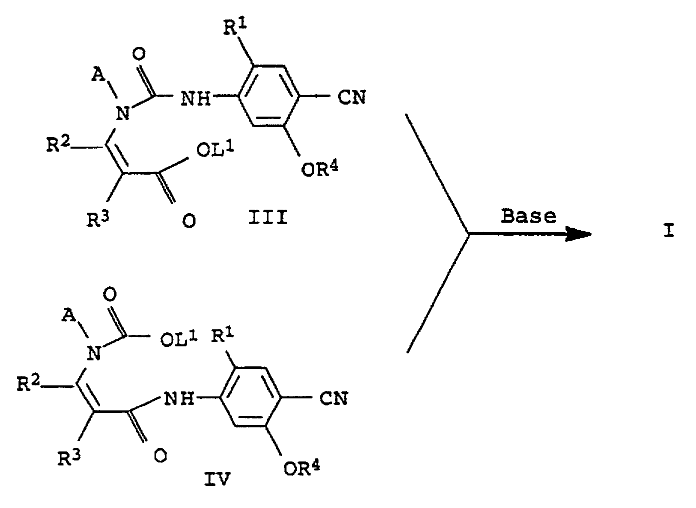

L¹ bedeutet niedermolekulares Alkyl, vorzugsweise C₁-C₄-Alkyl, oder Phenyl.

In der Regel cyclisiert man in einem inerten organischen Lösungs- oder Verdünnungsmittel, das aprotisch ist, beispielsweise in einem aliphatischen oder cyclischen Ether wie 1,2-Dimethoxyethan, Tetrahydrofuran und Dioxan, in einem Aromaten wie Benzol und Toluol oder in einem polaren Solvens wie Dimethylformamid und Dimethylsulfoxid. Auch Mischungen aus polarem Solvens und einem Kohlenwasserstoff wie n-Hexan sind geeignet. Je nach Ausgangsverbindung kann auch Wasser als Verdünnungsmittel geeignet sein.

Als Basen kommen vorzugsweise Alkalimetallalkoholate, insbesondere die Natriumalkoholate, Alkalimetallhydroxide, insbesondere Natriumhydroxid und Kaliumhydroxid, Alkalimetallcarbonate, insbesondere Natriumcarbonat und Kaliumcarbonat, und Metallhydride, insbesondere Natriumhydrid, in Betracht. Bei der Verwendung von Natriumhydrid als Base hat es sich als vorteilhaft erwiesen, in einem aliphatischen oder cyclischen Ether, in Dimethylformamid oder in Dimethylsulfoxid zu arbeiten.

Normalerweise ist die 0,5- bis 2-fache molaren Menge an Base, bezogen auf die Menge an III oder IV, für das Gelingen der Reaktion ausreichend.

Im allgemeinen liegt die Reaktionstemperatur bei (-78)°C bis Siedetemperatur des jeweiligen Reakionsgemisches, insbesondere bei (-60) bis 60°C.

Bedeutet A in Formel III oder IV Wasserstoff, so wird das Verfahrensprodukt als Metallsalz erhalten, wobei das Metall dem Kation der verwendeten Base entspricht. Das Salz kann auf an sich bekannte Weise isoliert und gereinigt oder gewnnschtenfalls mittels Säure in die freie Verbindung I mit A = Wasserstoff übergeführt werden.

### Verfahren B):

Methylierung einer Verbindung I, bei der A Wasserstoff bedeutet, in Gegenwart einer Base:

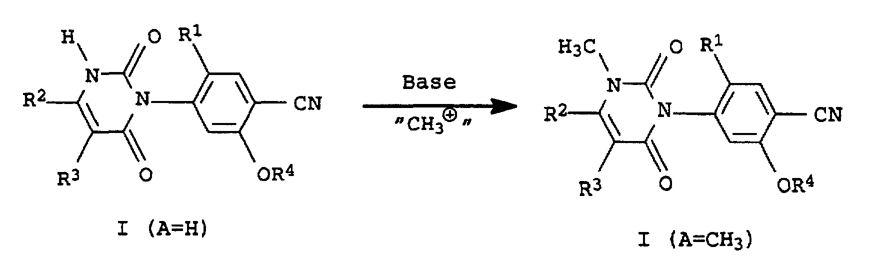

Als Methylierungsmittel kommen beispielsweise Methylhalogenide, vorzugsweise Methylchlorid, -iodid oder -bromid, sowie Dimethylsulfat, Methansulfonat (Methylmesylat), Methyl-benzolsulfonat, Methoxy-(p-toluylsulfon) (Methyltosylat), p-Brombenzolsulfonylmethan (Methylbrosylat), Trifluormethansulfonylmethan (Methyltriflat) und Diazomethan in Betracht.

In der Regel arbeitet man in einem inerten organischen Lösungsmittel oder in einem aprotischen Lösungsmittel, z.B. in einem aliphatischen oder cyclischen Ether, vorzugsweise in 1,2-Dimethoxyethan, Tetrahydrofuran oder Dioxan, in einem aliphatischen Keton, vorzugsweise in Aceton, in einem Amid, vorzugsweise in Dimethylformamid, in einem Sulfoxid, vorzugsweise in Dimethylsulfoxid, in einem Harnstoff wie Tetramethylharnstoff und 1,3-Dimethyltetrahydro-2(1H)-pyrimidinon, in einem Carbonsäureester wie Essigsäurethylester, oder in einem halogenierten aliphatischen oder aromatischen Kohlenwasserstoff wie Dichlormethan und Chlorbenzol.

Als Base eignen sich anorganische Basen, z.B. Carbonate wie Natriumcarbonat und Kaliumcarbonat, Hydrogencarbonate wie Natrium- und Kaliumhydrogencarbonat, oder Alkalimetallhydride wie Natriumhydrid und Kaliumhydrid, sowie organische Basen, z.B. Amine wie Triethylamin, Pyridin und N,N-Diethylanilin, oder Alkalimetallalkoholate wie Natriummethanolat, Natriumethanolat und Kalium-tert.-butanolat.

Die Menge an Base und Methylierungsmittel liegt vorzugsweise jeweils bei der 0,5- bis 2-fachen molaren Menge, bezogen auf die Menge an Ausgangsverbindung.

Im allgemeinen liegt die Reaktionstemperatur bei 0°C bis Siedetemperatur des Reaktionsgemisches, insbesondere bei 0 bis 60°C.

Eine bevorzugte Verfahrensvariante besteht darin, das aus der Cyclisierung von III (A = H) oder IV (A = H) gemäß Verfahren A) erhaltene Salz von I ohne Isolierung aus der Reaktionsmischung, die noch überschüssige Base, z.B. Natriumhydrid, Natriumalkoholat oder Natriumcarbonat, enthalten kann, zu methylieren.

Sofern nicht unmittelbar durch die als Methode a) beschriebene Cyclisierung unter basischen Bedingungen herstellbar, können die Salze derjenigen Verbindungen I, in denen A Wasserstoff bedeutet, auch in an sich bekannter Weise aus den Verfahrensprodukten der Methode a) erhalten werden. Zu diesem Zweck versetzt man beispielsweise die wässrige Lösung einer anorganischen oder organischen Base mit dem substituierten 3-(4-Cyanophenyl)uracil I, bei dem A für Wasserstoff steht. Die Salzbildung erfolgt normalerweise bereits bei 20-25°C mit ausreichender Geschwindigkeit.

Besonders vorteilhaft ist es, das Natriumsalz durch Auflösen des 3-(4-Cyanophenyl)uracils I (A = Wasserstoff), in wäßriger Natriumhydroxidlösung bei 20-25°C herzustellen, wobei etwa äquivalente Mengen an 3-(4-Cyanophenyl)uracil und Natriumhydroxid eingesetzt werden. Das Salz des 3-(4-Cyanophenyl)uracils kann dann z.B. durch Fällen mit einem geeigneten inerten Lösungsmittel oder durch Abdampfen des Lösungsmittels isoliert werden.

Salze der 3-(4-Cyanophenyl)uracile, deren Metallion kein Alkalimetallion ist, können üblicherweise durch Umsalzen des entsprechenden Alkalimetallsalzes in wässriger Lösung hergestellt werden. Auf diese Weise lassen sich z.B. 3-(4-Cyanophenyl)uracil-Metallsalze herstellen, die in Wasser unlöslich sind.

### Verfahren C):

Umsetzung eines 3-(4-Cyanophenyl)uracils der Formel I, wobei A Wasserstoff bedeutet, mit einem elektrophilen Aminierungsreagenz in Gegenwart einer Base:

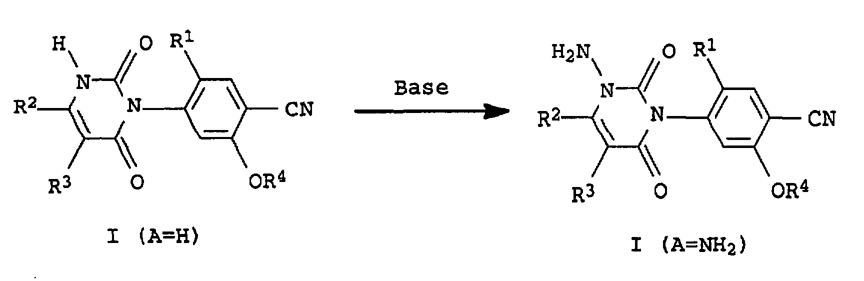

Als Aminierungsreagenz hat sich bisher 2,4-Dinitrophenoxyamin besonders bewährt, jedoch kann z.B. auch Hydroxylamin-O-sulfonsäure (HOSA) verwendet werden, die aus der Literatur bereits als Aminierungsreagenz bekannt ist (vgl. z.B. E. Hofer et al., Synthesis 1983, 466; W. Friedrichsen et al., Heterocycles 20 (1983) 1271; H. Hart et al., Tetrahedron Lett. 25 (1984) 2073; B. Vercek et al., Monatsh. Chem. 114 (1983) 789; G. Sosnousky et al., Z. Naturforsch. 38 (1983) 884; R.S. Atkinson et al., J. Chem. Soc. Perkin Trans. 1987, 2787).

Die Aminierung kann auf an sich bekannte Weise durchgeführt werden (siehe z.B. T. Sheradsky, Tetrahedron Lett. 1948, 1909; M.P. Wentland et al., J. Med. Chem. 27 (1984) 1103 und insbesondere EP-A 240 194, EP-A 476 697 und EP-A 517 181, wo die Aminierung von Uracilen gelehrt wird).

Normalerweise führt man die Umsetzung in einem polaren Lösungsmittel durch, z.B. in Dimethylformamid, N-Methylpyrrolidon, Dimethylsulfoxid oder in Ethylacetat, das sich bisher als besonders geeignet erwiesen hat.

Als Base eignen sich beispielsweise Alkalimetallcarbonate wie Kaliumcarbonat, Alkalimetallalkoholate wie Natriummethylat und Kalium-tert.-butanolat oder Alkalimetallhydride wie Natriumhydrid.

Die Menge an Base und Aminierungsmittel liegt vorzugsweise jeweils bei der 0,5- bis 2-fachen molaren Menge, bezogen auf die Menge an Ausgangsverbindung.

Je nach Bedeutung von R⁴ kann es erforderlich sein, diesen Substituenten vor der Aminierung in an sich bekannter Weise zu schützen. Dies ist besonders empfehlenswert, wenn R⁴ für Wasserstoff steht.

### Verfahren D):

Etherspaltung eines 3-(4-Cyanophenyl)uracils der Formel I, bei dem R⁴ Alkyl, Cycloalkyl, Alkenyl oder Alkinyl bedeutet:

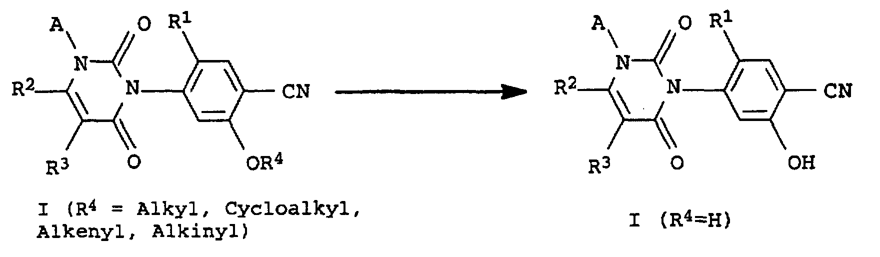

Die Etherspaltung erfolgt üblicherweise mittels Säure, z.B. mittels Bromwasserstoff, Iodwasserstoff oder Pyridiniumhydrochlorid, mittels einer Lewis-Säure wie Aluminiumtrichlorid, -tribromid, -triiodid, Bortrichlorid, -tribromid, -trifluorid und Eisentrichlorid, oder mittels Trimethylsilyliodid. Daneben sind aber auch Lithiumsalze wie Lithiumchlorid oder Mischungen aus einem anorganischen Iodid und Trimethylsilylchlorid brauchbar, um die Etherbindung zu spalten. In Einzelfällen kann die Bindung auch unter Hydrierbedingungen mittels Wasserstoff in Gegenwart eines Hydrierkatalysators wie Platin und Palladium auf Aktivkohle gespalten werden.

Allylether (R⁴ = Allyl) können ferner auf hierfür an sich bekannte Weise in die entsprechenden Phenole übergeführt werden, z.B. durch Isomerisierung in Gegenwart eines Übergangsmetallkatalysators zum Enolether und Spaltung von Letzterem, vorzugsweise unter leicht sauren Bedingungen (vgl. z.B. T. Greene u. P.G.M. Wutz in "Protective Groups in Organic Synthesis", John Wiley & Sons, 2. Auflage New York 1991, S. 42ff.).

Üblicherweise arbeitet man in einem inerten Lösungs- oder Verdünnungsmittel, z. B. in einem aliphatischen, cyclischen oder aromatischen Kohlenwasserstoff wie n-Pentan, Petrolether, Cyclohexan, Benzol, Toluol oder Xylol, einem aliphatischen oder cyclischen Ether wie Diethylether, tert.-Butylmethylether, Dimethoxyethan und Tetrahydrofuran, einem aliphatischen oder aromatischen Halogenkohlenwasserstoff wie Dichlormethan, Chloroform, Chorbenzol, 1,2-Dichlorethan und den Dichlorbenzolen, einem Alkohol wie Methanol, Ethanol und tert.-Butanol, einem Amid wie Dimethylformamid und N-Methylpyrrolidon, einem Amin wie Ammoniak, oder in einem Gemisch derartiger Solventien.

Eine Reaktionsführung ohne Lösungsmittel kann auch vorteilhaft sein.

Bezüglich besonders bevorzugter Ausführungsformen sei auf die Ausführungen in Houben-Weyl, "Methoden der Organischen Chemie", Georg Thieme Verlag, 4. Auflage, Stuttgart 1979, Bd. 6/1a/1, S. 309ff und in R. C. Larock, "Comprehensive Organic Transformations", VCH-Publishers, Weinheim 1989, S. 501ff sowie auf die dort zitierte Literatur verwiesen.

### Verfahren E):

Alkylierung eines 3-(4-Cyanophenyl)uracils der Formel I, bei dem R⁴ Wasserstoff bedeutet, in Gegenwart einer Base:

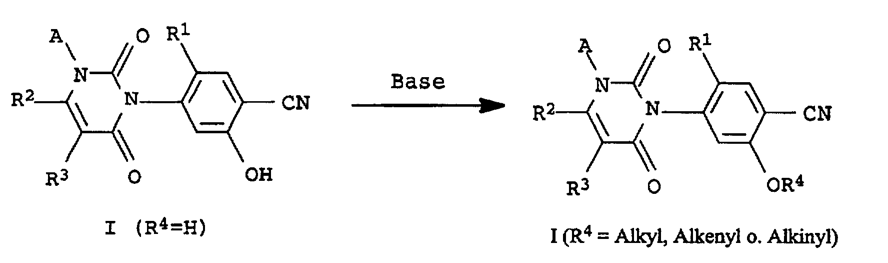

Die Alkylierung kann beispielsweise mit dem Halogenid, vorzugsweise dem Chlorid oder Bromid, dem Sulfat, Sulfonat, vorzugaweise dem Methansulfonat (Mesylat), Benzolsulfonat, p-Toluolsulfonat (Tosylat), p-Brombenzolsulfonat (Brosylat), dem Trifluormethansulfonat (Triflat) oder der Diazoverbindung eines unsubstituierten oder substituierten Alkans, Cycloalkans, Halogenalkans, Alkens oder Alkins vorgenommen werden.

Normalerweise arbeitet man in einem inerten organischen Lösungsmittel, wobei besonders aprotische Lösungsmittel, z.B. aliphatische und cyclische Ether wie 1,2-Dimethoxyethan, Tetrahydrofuran und Dioxan, aliphatische Ketone wie Aceton, Amide wie Dimethylformamid, Sulfoxide wie Dimethylsulfoxid, Harnstoffen wie Tetramethylharnstoff und 1,3-Dimethyltetrahydro-2(1H)-pyrimidinon, Carbonsäureester wie Essigsäureethylester, oder halogenierte aliphatische oder aromatische Kohlenwasserstoffe wie Dichlormethan und Chlorbenzol, in Betracht kommen.

Als Base eignen sich sowohl anorganische Basen, z.B. Alkalimetallcarbonate wie Natriumcarbonat und Kaliumcarbonat, Alkalimetallhydrogencarbonate wie Natrium- und Kaliumhydrogencarbonat, oder Alkalimetallhydride wie Natriumhydrid und Kaliumhydrid, als auch organische Basen, z.B. Amine wie Triethylamin, Pyridin und N,N-Diethylanilin, oder Alkalimetallalkoholate wie Natriummethanolat, -ethanolat und Kalium-tert.-butanolat.

Die Menge an Base und Alkylierungsmittel liegt vorzugsweise bei der 0,5- bis 2-fachen molaren Menge, bezogen auf die Menge an I mit R⁴ = Wasserstoff.

Im allgemeinen empfiehlt sich eine Reaktionstemperatur von 0°C bis zur Siedetemperatur des Reaktionsgemisches, insbesondere von 0 bis 60°C.

Etwaige Regioselektivitätsprobleme bei Ausgangsverbindungen mit A = Wasserstoff können auf an sich bekannte Weise (Verwendung von 2 Äquivalenten Base, Einführung einer Schutzgruppe etc.) vermieden werden.

### Verfahren F):

Acylierung eines 3-(4-Cyanophenyl)uracils der Formel I, wobei R⁴ Wasserstoff bedeutet, mit einem geeigneten Acylierungsmittel.

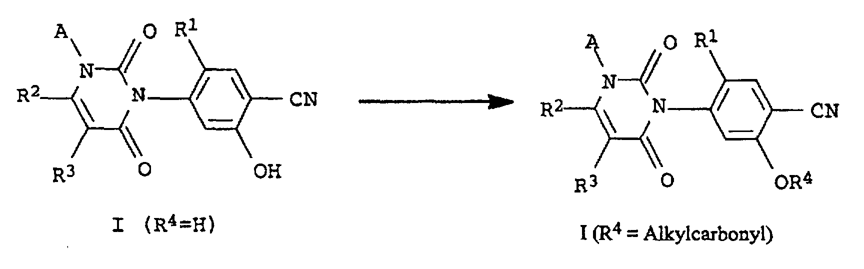

Geeignete Acylierungsmittel sind z.B. die Säurehalogenide, insbesondere die Säurechloride, die Anhydride, Isocyanate oder Sulfonylchloride von Alkancarbonsäuren. Es kommen aber auch die freien Säuren oder deren Anhydride in Betracht, sofern dann in Gegenwart eines Kondensationsmittel wie Carbonyldiimidazol und Dicyclohexylcarbondiimid gearbeitet wird.

In der Regel arbeitet man in einem inerten organischen Lösungs- oder Verdünnungsmittel, das vorzugsweise aprotisch ist, z.B. in einem aliphatischen oder cyclischen Ether wie 1,2-Dimethoxyethan, Tetrahydrofuran und Dioxan, einem aliphatischen Keton wie Aceton, einem Amid wie Dimethylformamid, einem Harnstoff wie Tetramethylharnstoff und 1,3-Dimethyltetrahydro2(1H)-pyrimidinon, einem Carbonsäureester wie Essigsäurethylester, oder einem aliphatischen oder aromatischen Halogenkohlenwasserstoff wie Dichlormethan und Chlorbenzol.

Bezüglich geeigneter Basen, der Mengenverhältnisse und der Reaktionstemperatur sei auf die Ausführungen unter Verfahren E) verwiesen.

### Verfahren G):

Substitution von Halogenid durch Cyanid:

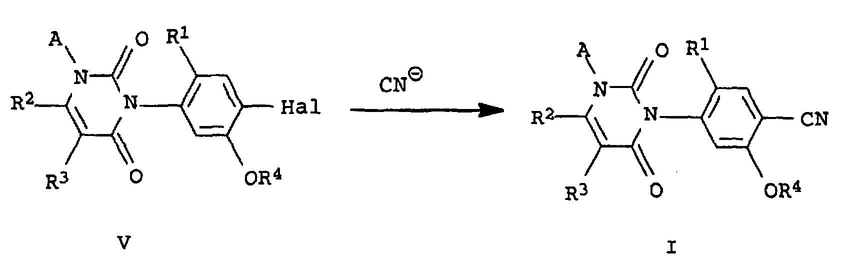

Hal steht für Halogen, vorzugsweise für Fluor, Brom oder Jod.

Geeignete Cyanide sind insbesondere Metallcyanide, z.B. die Alkalimetallcyanide wie Lithium-, Natrium- und Kaliumcyanid, die Erdalkalimetallcyanide wie Magnesiumcyanid, oder auch Übergangsmetallcyanide wie Kupfercyanid.

Üblicherweise arbeitet man in einem Ether wie Tetrahydrofuran, Dioxan und 1,2-Dimethoxyethan, oder in einem aprotischen, polaren Lösungsmittel, z.B. einem Alkylnitril wie Aceto-, Propio- und Butyronitril, einem Alkylharnstoff wie N,N,N',N'-Tetramethylharnstoff, einem offenkettigen oder cyclischen Dialkylamid wie Dimethylformamid, N-Methyl-2-pyrrolidon, 1,2-Dimethyl-imidazolidin-2-on und 1,2-Dimethyl-3,4,5,6-tetrahydro-2(1H)-pyrimidinon, einem Dialkylsulfoxid wie Dimethylsulfoxid, oder in Hexamethylphosphorsäuretriamid.

Nach den bisherigen Erkenntnissen kann sich die Gegenwart eines Katalysators vorteilhaft auf den Reaktionsverlauf auswirken. Brauchbare Katalysatoren sind z.B. Übergangsmetalle und deren Komplexe oder Salze, z.B. Verbindungen des Kupfers wie Kupfer-(I)-chlorid, -iodid, -cyanid, oder des Nickels wie Nickel-bis-triphenylphospin-dibromid.

Bei Ausgangsverbindungen V mit A = Wasserstoff empfiehlt es sich, in Gegenwart einer Base zu arbeiten, wobei insbesondere schwach nucleophile Basen in Betracht kommen, und zwar sowohl anorganische Basen, z.B. Alkalimatellcarbonate wie Natrium- und Kaliumcarbonat, Alkalimetallhydrogencarbonate wie Natrium- und Kaliumhydrogencarbonat, oder Alkalimetallhydride wie Natriumhydrid und Kaliumhydrid, als auch organische Basen, z.B. Amine wie Triethylamin, Pyridin und N,N-Diethylanilin.

Die Mengenverhältnisse sind normalerweise nicht kritisch. Im allgemeinen ist die etwa ein- bis 10-fache Menge an Cyanid und Base, bezogen auf die Menge an V, ausreichend.

Die Reaktionstemperatur liegt üblicherweise bei 50 bis 250°C; zur Erhöhung der Selektivität der Reaktion kann es aber auch empfehlenswert sein, bei tieferen Temperaturen, insbesondere bei etwa 20°C, zu arbeiten.

Bezüglich verschiedener Ausführungsformen dieser Umsetzung sei auf Houben-Weyl, "Methoden der Organischen Chemie", Georg Thieme Verlag, 4. Auflage, Stuttgart 1985, Bd. E5, S. 1444ff. sowie auf die dort angegebene Literatur verwiesen.

### Verfahren H):

Halogenierung eines 3-(4-Cyanophenyl)uracils der Formel I, bei dem R³ Wasserstoff bedeutet

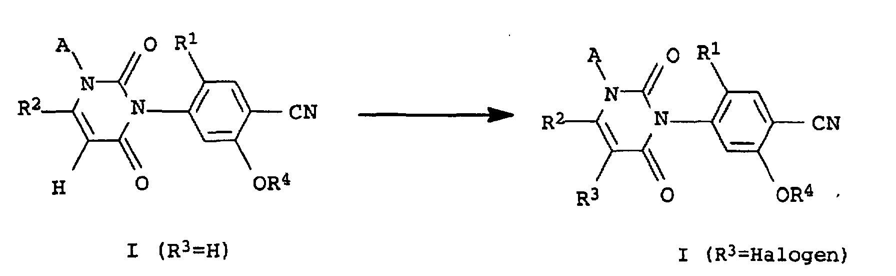

Die Halogenierung erfolgt in der Regel in einem inerten organischen Lösungs- oder Verdünnungsmittel. Für die Chlorierung und Bromierung kommen beispielsweise aliphatische Carbonsäuren wie Essigsäure, oder chlorierte aliphatische Kohlenwasserstoffe wie Methylenchlorid, Chloroform und Tetrachlorkohlenstoff, in Betracht. Für die Jodierung sind niedrig siedendende aliphatischen Carbonsäuren wie Essigsäure besonders bevorzugt.

Für die Chlorierung und Bromierung eignen sich besonders elementares Chlor bzw. Brom, oder Sulfurylchlorid bzw. Sulfurylbromid, bei einer Reaktionstemperatur von vorzugsweise 0 bis 60°C, insbesondere 10 bis 30°C.

Gewünschtenfalls kann die Chlorierung und Bromierung in Gegenwart eines säurebindenden Mittels erfolgen, wobei Natriumacetat und tertiäre Amine wie Triethylamin, Dimethylanilin und Pyridin besonders bevorzugt sind.

Als Jodierungsmittel ist elementares Jod besonders bevorzugt, wobei in diesem Fall die Reaktionstemperatur bei ca. 0 bis 110°C, vorzugsweise bei 10 bis 30°C, liegt.

Besonders vorteilhaft ist die Jodierung in Gegenwart einer Mineralsäure wie rauchende Salpetersäure.

Die Menge an Halogenierungsmittel ist nicht kritisch; normalerweise verwendet man äquimolare Mengen an Halogenierungsmittel oder einen Überschuß bis etwa 200 mol-%, bezogen auf das zu halogenierende Edukt.

Überschüssiges Jod kann beispielsweise nach der Reaktion mittels gesättigter wäßriger Natriumhydrogensulfitlösung entfernt werden.

### Verfahren I):

Substitution der Nitrogruppe von 3-(4-Cyano-3-nitrophenyl)uracilen VI durch eine Gruppe -OR⁴:

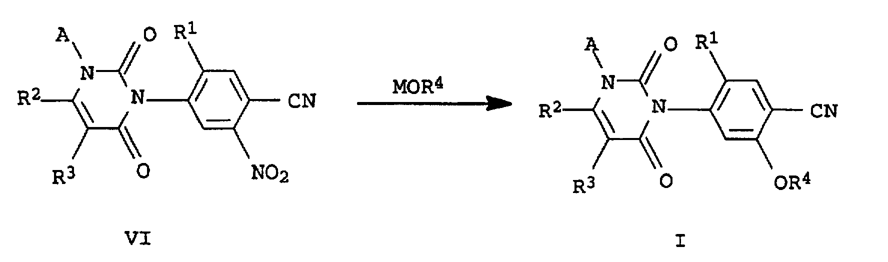

Die Substitution der Nitrogruppe erfolgt üblicherweise durch Umsetzung von VI mit einem Alkoholat MOR⁴, wobei M für ein Metallatom, vorzugsweise Lithium, Natrium oder Kalium steht (vgl. z.B. Org. Synth. Coll. Vol. III, 293).

In der Regel arbeitet man entweder in dem Alkohol HOR⁴, dessen Alkoholat verwendet wird, oder in einem inerten organischen Lösungs- oder Verdünnungsmittel, z.B. in einem aromatischen Kohlenwasserstoff wie Toluol und die Xylole, in einem Ether wie Diethylether, Tetrahydrofuran und 1,2-Dimethoxyethan, oder in einem halogenierten Kohlenwasserstoff wie Dichlormethan und Chlorbenzol.

Die Reaktionstemperatur liegt im allgemeinen bei 0 bis 150°C, vorzugsweise bei Raumtemperatur (etwa 20°C) bis zur Siedetemperatur des jeweiligen Reaktionsgemisches.

Die Menge an Alkoholat ist normalerweise nicht kritisch; bevorzugt sind etwa 1 bis 3 Äquivalente Alkoholat pro Mol VI.

Die 3-(4-Cyano-3-nitrophenyl)uracile V sind ihrerseits z.B. aus 3-(4-Halogen-3-nitrophenyl)uracilen VII 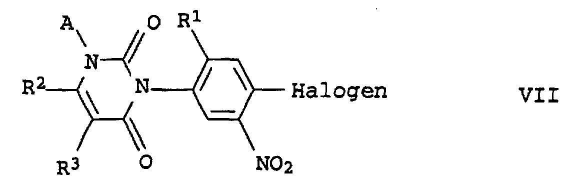 erhältlich, indem man das Halogen durch Cyano substituiert.
Die bei Verfahren G) gemachten Angaben gelten hierbei entsprechend.

Die 3-(4-Halogen-3-nitrophenyl)uracile VII wiederum lassen sich z.B. durch Nitrierung von 3-(4-Halogenphenyl) uracilen VIII 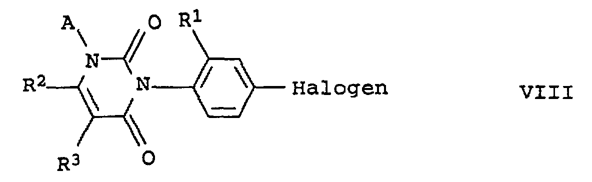 mit Salpetersäure, Nitriersäure, mit einem anorganischen Nitrat wie Natrium-, Kalium- und Ammoniumnitrat oder mit einem organische Nitrat wie Amylnitrat, herstellen.

Geeignete Lösungsmittel für die Nitrierung sind vorzugsweise anorganische Säuren wie Salpetersäure und Schwefelsäure, organische Säuren wie Essigsäure, oder Anhydride wie Essigsäureanhydrid.

Die Reaktionstemperatur liegt normalerweise bei (-20) bis 50°C, vorzugsweise bei (-10) bis 30°C.

Die Menge an Nitrierungsmittel ist nicht kritisch; sie liegt normalerweise bei der ein- bis 10-fachen molaren Menge, bezogen auf die Menge an VI.

### Verfahren K):

Überführung von 3-(4-Aminophenyl)uracilen IX in Verbindungen I nach der Methode von Sandmeyer:

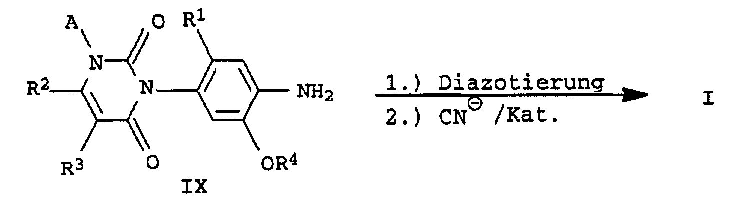

Bei diesem Reaktionstyp geht man üblicherweise so vor, daß man die Aminogruppe auf an sich bekannte Weise in das Diazoniumsalz überführt, und dieses anschließend in Gegenwart eines Übergangsmetallkatalysators, insbesondere eines Kupfer-(I)-salzes, zweckmäßigerweise Kupfer(I)cyanid, mit einem Metallcyanid, vorzugsweise mit Lithium-, Natrium- oder Kaliumcyanid, umsetzt.

Bezüglich der Verfahrensbedingungen sei beispielsweise auf die Ausführungen in C. Ferri, "Reaktionen der organischen Chemie", Georg Thieme Verlag, Stuttgart 1978, S. 319 und in Organic Synthesis Coll. Vol 1, S 514 (1941) verwiesen.

Die Ausgangsverbindungen IX sind vorzugsweise durch Reduktion der entsprechenden Nitroverbindungen mit Wasserstoff in Gegenwart eines Metallkatalysators, der aus Raney-Nickel, Palladium oder Platin besteht, oder mit einem Reduktionsmittel, z.B. einem Zinn-II-salz oder Eisen, herstellbar. Weitere Angaben zu dieser an sich bekannten Reaktion sind beispielsweise der DE-A 37 24 399 zu entnehmen.

Die den Verbindungen IX entsprechenden, nitrierten Vorprodukte wiederum sind zweckmäßigerweise durch Nitrierung von Phenylverbindungen X 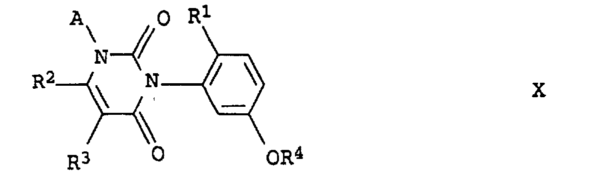 erhältlich. Die bezüglich der Nitrierung der 3-(4-Halogenphenyl)uracilen VII bei Verfahren I) gemachten Angaben gelten entsprechend für die Nitrierung der Phenyluracile X.

### Verfahren L):

Direkte Cyanierung einer Phenylverbindung X:

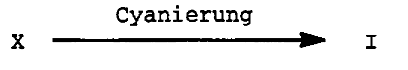

Die Cyanierung kann ohne Lösungsmittel oder in einem inerten Lösungs- oder Verdünnungsmittel vorgenommen werden, beispielsweise in einem aliphatischen, cyclischen oder aromatischen Kohlenwasserstoff wie n-Pentan, Petrolether und Cyclohexan, einem aliphatischen oder cyclischen Ether wie Diethylether, tert.-Butylmethylether, Dimethoxyethan und Tetrahydrofuran, einem aliphatischen oder aromatischen Halogenkohlenwasserstoff wie Dichlormethan, Chloroform, 1,2-Dichlorethan und den Dichlorbenzolen, einem Alkohol wie Ethanol, Methanol und tert.-Butanol, einem Amid wie Dimethylformamid und N-Methylpyrrolidon, oder einem Amin wie Ammoniak. Auch Mischungen derartiger Solventien kommen in Betracht.

Als Cyanid-Quelle eignen sich Alkylthiocyanate wie Methylthiocyanat {vgl. z.B. Synth. Commun. 20, 71 (1990)}, Chlorsulfonylisocyanat (vgl. z.B. Org. Synth. Coll. Vol VI, S. 465), Dicyan, Chlorcyan und Bromcyan, des weiteren Trichloracetonitril {vgl. hierzu Gazz. Chim. Ital. 122, 283 (1992)}.

Die Temperaturen liegen normalerweise bei (-20) bis 150°C, bevorzugt bei (-10)°C bis Siedetemperatur des jeweiligen Reaktionsgemisches.

Das Verhältnis Cyanierungsmittel zu IX ist nicht kritisch; es liegt normalerweise bei 1:1 bis 10:1.

Modifikationen dieser Umsetzung sind u.a. in Houben-Weyl, "Methoden der Organischen Chemie", Georg Thieme Verlag, Bd. E5, 4. Auflage, Stuttgart 1985, S. 1447 ff und in der dort zitierten Literatur beschrieben.

Die Ausgangsverbindungen der Formel VI, VII, VIII und IX sind bekannt oder auf an sich bekannte Weise herstellbar (vgl. z.B. EP-A 255 047, EP-A 517 181 und JP-A 05/025 143).

Die Enamin-Ester der Formel III sind neu. Sie können ebenfalls als Herbizide eingesetzt werden.

Ihre Herstellung kann nach an sich bekannten Methoden erfolgen, z. B. nach einem der folgenden Verfahren:
M) :

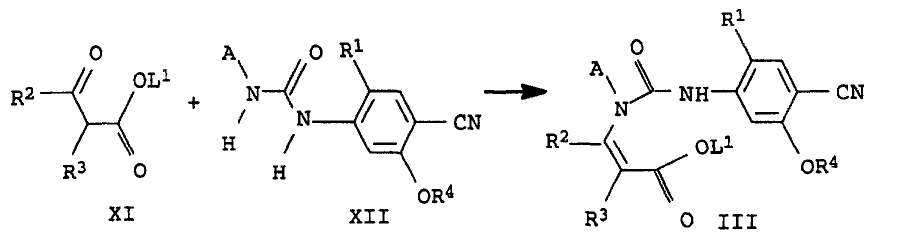

Vorzugsweise arbeitet man im wesentlichen wasserfrei in einem inerten Lösungs- oder Verdünnungsmittel, besonders bevorzugt in Gegenwart eines sauren oder basischen Katalysators.

Als Lösungs- oder Verdünnungsmittel kommen insbesondere mit Wasser azeotrop mischbare organische Lösungsmittel, beispielsweise Aromate wie Benzol, Toluol und o-, m-, p-Xylol, halogenierte Kohlenwasserstoffe wie Methylenchlorid, Chloroform, Tetrachlorkohlenstoff und Chlorbenzol, aliphatische und cyclische Ether wie 1,2-Dimethoxyethan, Tetrahydrofuran und Dioxan, oder Cyclohexan, aber auch Alkohole wie Methanol und Ethanol, in Betracht.

Als saure Katalysatoren eignen sich bevorzugt starke Mineralsäuren wie Schwefelsäure und Salzsäure, Phosphor enthaltende Säuren wie Orthophosphorsäure und Polyphosphorsäure, organische Säuren wie p-Toluolsulfonsäure sowie saure Kationenaustauscher wie "Amberlyst 15" (Fluka).

Als basische Katalysatoren eignen sich z.B. Metallhydride wie Natriumhydrid sowie besonders bevorzugt Metallalkoholate wie Natriummethanolat und Ethanolat.

Zweckmäßig setzt man XI und den β-Ketoester XII in etwa stöchiometrischem Verhältnis ein oder man arbeitet mit einem geringen Überschuß der einen oder anderen Komponente, bis etwa 10 mol-%.

Normalerweise ist eine Katalysatormenge von 0,5 bis 2 mol-%, bezogen auf die Menge eines Edukts, ausreichend.

Im allgemeinen erfolgt die Reaktionsführung bei einer Temperatur von 60 bis 120°C, zur raschen Entfernung von entstehendem Wasser vorzugsweise bei der Siedetemperatur des Reaktionsgemisches.
N) :

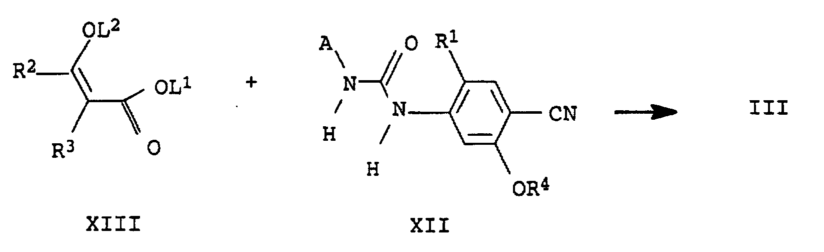

L² bedeutet C₁-C₄-Alkyl oder Phenyl.

Diese Umsetzung kann beispielsweise in einem inerten, mit Wasser mischbaren, organischen Lösungsmittel, beispielsweise einem aliphatischen oder cyclischen Ether wie 1,2-Dimethoxyethan, Tetrahydrofuran und Dioxan, oder einem niederen Alkohol, insbesondere Ethanol, durchgeführt werden, wobei die Reaktionstemperatur normalerweise bei 50 bis 100°C, vorzugsweise bei der Siedetemperatur des Reaktionsgemisches, liegt.

Die Reaktion kann jedoch auch in einem aromatischen Verdünnungsmittel wie Benzol, Toluol und o-, m-, p-Xylol durchgeführt werden, wobei in diesem Fall der Zusatz entweder eines sauren Katalysators wie Salzsäure und p-Toluolsulfonsäure oder einer Base, z.B. eines Alkalimetallalkoholates wie Natriummethanolat und Natriumethanolat, empfehlenswert ist. Auch bei dieser Verfahrensvariante liegt die Reaktionstemperatur normalerweise bei 50 bis 100°C, bevorzugt jedoch bei 60 bis 80°C.

Bezüglich der Mengenverhältnisse gelten die Angaben für Methode M).
O) :

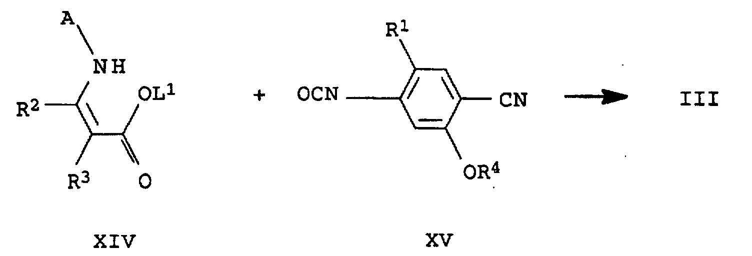

Die Umsetzung erfolgt zweckmäßig in Gegenwart eines im wesentlichen wasserfreiem aprotischen organischen Lösungs- oder Verdünnungsmittel, beispielsweise eines aliphatischen oder cyclischen Ethers wie Diethylether, 1,2-Dimethoxyethan, Tetrahydrofuran und Dioxan, eines aliphatischen oder aromatischen Kohlenwasserstoffs wie n-Hexan, Benzol, Toluol und o-, m-, p-Xylol, eines halogenierten, aliphatischen Kohlenwasserstoffs wie Methylenchlorid, Chloroform, Tetrachlorkohlenstoff, 1,2-Dichlorethan und Chlorbenzol, eines aprotischen, polaren Lösungsmittel wie Dimethylformamid, Hexamethylphosphorsäuretriamid und Dimehylsulfoxid, oder eines Gemisches aus den genannten Solventien.

Gewünschtenfalls kann auch in Gegenwart einer Metallhydridbase wie Natrium- und Kaliumhydrid oder einer organischen tertiären Base wie Triethylamin und Pyridin gearbeitet werden, wobei die organische Base gleichzeitig als Lösungsmittel dienen kann.

Zweckmäßig setzt man die Edukte in stöchiometrischem Verhältnis ein oder man arbeitet mit einem geringen Überschuß der einen oder anderen Komponente bis etwa 10 mol-%. Arbeitet man ohne Lösungsmittel in Gegenwart einer organischen Base, so liegt diese in einem größeren Überschuß vor.

Die Reaktionstemperatur liegt vorzugsweise bei (-80) bis 50°C, insbesondere bei (-60) bis 30°C.

In einer besonders bevorzugten Ausführungsform wird der erhaltene Enamin-Ester III mit überschüssiger Base direkt (d.h. "in situ") gemäß Verfahren A) in das entsprechende Wertprodukt I übergeführt.
P) :

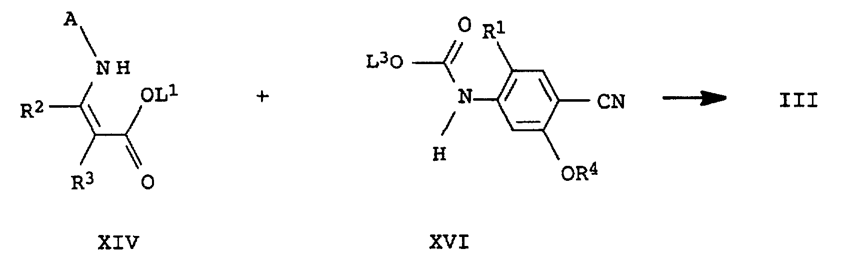

L³ bedeutet C₁-C₄-Alkyl oder Phenyl.

Diese Reaktion erfolgt zweckmäßig in einem aprotischen, polaren Lösungs- oder Verdünnungsmittel wie Dimethylformamid, 2-Butanon, Dimethylsulfoxid und Acetonitril, und zwar vorteilhaft in Gegenwart einer Base, beispielsweise eines Akalimetall- oder Erdalkalimetallalkoholats, insbesondere eines Natriumalkanolates wie Natriummethanolat, eines Akalimetall- oder Erdalkalimetallcarbonates, insbesondere Natriumcarbonat, oder eines Alkalimetallhydrids wie Lithium- und Natriumhydrid.

Normalerweise ist die 1- bis 2-fache molare Menge an Base, bezogen auf die Menge von XIV oder XVI, ausreichend.

Die Reaktionstemperatur liegt im allgemeinen bei 80 bis 180°C, vorzugsweise bei der Siedetemperatur des Reaktionsgemisches.

Bezüglich der Mengenverhältnisse der Ausgangsverbindungen gelten die Angaben für Methode M).

In einer besonders bevorzugten Ausführungsform verwendet man ein Natriumalkoholat als Base und destilliert den im Laufe der Reaktion entstehenden Alkohol kontinuierlich ab. Die auf diese Weise hergestellten Enamin-Ester III können ohne Isolierung aus der Reaktionsmischung gemäß Verfahren A) zu einem Salz der substituierten 3-(4-Cyanophenyl)urazile I cyclisiert werden.
Q):

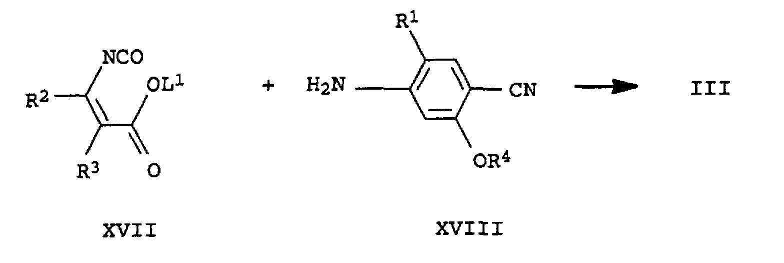

Diese Umsetzung erfolgt zweckmäßig in einem im wesentlichen wasserfreien, aprotischen, organischen Lösungs- oder Verdünnungsmittel, beispielsweise in Gegenwart eines aliphatischen oder cyclischen Ethers wie Diethylether, 1,2-Dimethoxyethan, Tetrahydrofuran und Dioxan, eines aliphatischen oder aromatischen Kohlenwasserstoffs wie n-Hexan, Benzol, Toluol und o-, m-, p-Xylol, eines halogenierten, aliphatischen Kohlenwasserstoffs wie Methylenchlorid, Chloroform, Tetrachlorkohlenstoff, 1,2-Dichlorethan und Chlorbenzol, eines aprotischen, polaren Lösungsmittels wie Dimethylformamid, Hexamethylphosphorsäuretriamid und Dimethylsulfoxid, oder eines Gemisches aus den genannten Solventien.

Gewünschtenfalls kann in Gegenwart einer Metallhydridbase wie Natrium- und Kaliumhydrid, eines Alkalimetall- oder Erdalkalimetallalkoholates wie Natriummethanolat, -ethanolat und Kalium-tert.-butanolat, oder einer organischen Stickstoffbase wie Triethylamin und Pyridin gearbeitet werden, wobei die organische Base gleichzeitig als Lösungsmittel dienen kann.

Zweckmäßig setzt man die Edukte in stöchiometrischen Mengen ein oder man verwendet eine der Komponenten im Überschuß, bis etwa 20 mol-%. Arbeitet man ohne Lösungsmittel in Gegenwart einer organischen Base, so liegt diese vorteilhaft in einem noch größeren Überschuß vor.

Die Reaktionstemperatur liegt im allgemeinen bei (-80) bis 150°C, vorzugsweise bei (-30)°C bis Siedetemperatur des jeweiligen Reaktionsgemisches.

Die Enamin-Carboxylate der Formel IV sind ebenfalls neu; auch sie können auf an sich bekannte Weise hergestellt werden, beispielsweise aus einem Anilinderivat der Formel VIII nach folgendem Reaktionsschema:
(G11) 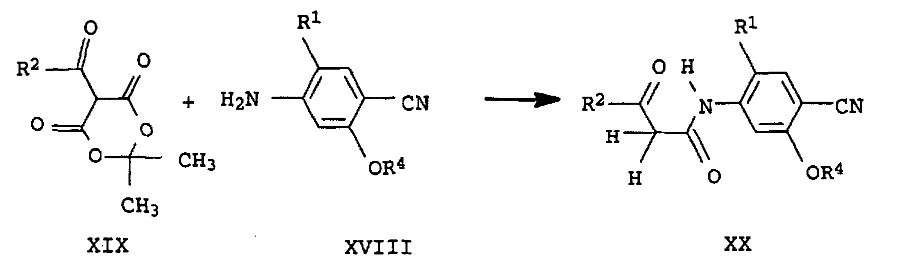 (G12)

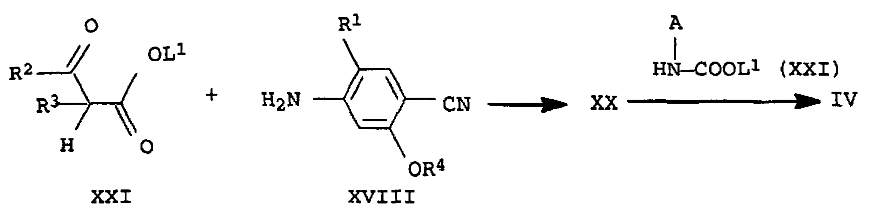

Die Umsetzung nach Reaktionsgleichung 1 erfolgt vorzugsweise in einem wasserfreien inerten aprotischen Lösungsmittel, beispielsweise in einem halogenierten Kohlenwasserstoff wie Methylenchlorid, Chloroform, Tetrachlorkohlenstoff und Chlorbenzol, einem aromatischen Kohlenwasserstoff wie Benzol, Toluol und o-, m-, p-Xylol, oder einem aliphatischen oder cyclischen Ether wie Diethylether, Dibutylether, 1,2-Dimethoxyethan, Tetrahydrofuran und Dioxan.

Bei der Umsetzung von XIX mit XVIII entsprechend Gleichung (G11) liegt die Reaktionstemperatur im allgemeinen bei etwa 70 bis 140°C, insbesondere bei 100 bis 120°C.

Bei der Umsetzung nach Reaktionsgleichung (G12) handelt es sich um eine Aminolyse, die in der Regel entweder ohne Lösungsmittel [vgl. z.B. J. Soc. Dyes Col. 42, 81 (1926), Ber. 64, 970 (1931); Org. Synth., Coll. Vol. IV, 80 (1943) und J.A.C.S. 70, 2402 (1948)] oder in einem inerten wasserfreien Lösungs-/Verdünnungsmittel, insbesondere in einem aprotischen Solvens, beispielsweise in einem Aromaten wie Toluol und o-, m-, p-Xylol oder einem halogenierten Aromaten wie Chlorbenzol, durchgeführt wird.

Hierbei empfiehlt sich das Arbeiten in Gegenwart eines basischen Katalysators, beispielsweise eines höher siedenden Amins [siehe z. B. Helv. Chim. Acta 11, 779 (1928) und U.S. 2,416,738] oder Pyridin.

Vorzugsweise liegt die Reaktionstemperatur bei ca. 130 bis 160°C.

Bei beiden Umsetzungen {(G11) und (G12)} setzt man die Ausgangsverbindungen zweckmäßigerweise in etwa stöchiometrischen Mengen ein oder man arbeitet mit einem geringen Überschuß der einen oder anderen Komponente bis etwa 10 mol-%. Arbeitet man in Gegenwart eines basischen Katalysators, so ist normalerweise eine Katalysatormenge von 0,5 bis 2 mol-%, bezogen auf die Menge eines der Edukte, ausreichend.

Die anschließende Umsetzung der so hergestellten Verbindungen der Formel XX mit dem Amin XXI wird vorteilhaft in einem weitgehend wasserfreien Lösungs-/Verdünnungsmittel bei Normaldruck durchgeführt, besonders bevorzugt in Gegenwart eines sauren Katalysators.

Zur Herstellung von Enamincarboxylaten IV mit A = Amino empfiehlt es sich, Verbindungen XXI mit geschützter Aminogruppe (z.B. als Hydrazon) einzusetzen.

Als Lösungs-/Verdünnungsmittel kommen insbesondere mit Wasser azeotrop mischbare organische Flüssigkeiten, beispielsweise Aromaten wie Benzol, Toluol und o-, m-, p-Xylol oder halogenierte Kohlenwasserstoffe wie Tetrachlorkohlenstoff und Chlorbenzol, in Betracht.

Geeignete Katalysatoren sind insbesondere starke Mineralsäuren wie Schwefelsäure, organische Säuren wie p-Toluolsulfonsäure, Phosphor enthaltende Säuren wie Orthophosphorsäure und Polyphosphorsäure oder saure Kationenaustauscher wie "Amberlyst 15" (Fluka).

Im allgemeinen liegt die Reaktionstemperatur bei etwa 70 bis 150°C; zur raschen Entfernung des entstehenden Reaktionswassers arbeitet man jedoch zweckmäßigerweise bei der Siedetemperatur des jeweiligen Reaktionsgemisches.

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Die Aufarbeitung der Reaktionsgemische erfolgt in der Regel nach an sich bekannten Methoden, beispielsweise durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die 3-(4-Cyanophenyl)uracile der Formel I können ein oder mehrere Chiralitätszentren enthalten und fallen dann üblicherweise als Enantiomeren- oder Diastereomerengemische an. Die Mischungen können gewünschtenfalls nach den hierfür üblichen Methoden, z.B. mittels Kristallisation oder Chromatographie an einem optisch aktiven Adsorbat, in die weitgehend reinen Isomeren getrennt werden. Reine optisch aktive Isomere lassen sich beispielsweise auch aus entsprechenden optisch aktiven Ausgangsmaterialien herstellen.

3-(4-Cyanophenyl)uracile I, bei denen A Wasserstoff bedeutet, lassen sich auf an sich bekannte Weise in ihre Salze, vorzugsweise in ihre Alkalimetallsalze, überführen (vgl. hierzu auch Herstellungsmethode b)).

Salze von I, deren Metallion kein Alkalimetallion ist, können durch Umsalzen des entsprechenden Alkalimetallsalzes in üblicher Weise hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Des weiteren eignen sich die 3-(4-Cyanophenyl)uracile I auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden Mittel können beispielsweise in Form von direkt versprühbaren waßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder öldispersionen kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Im allgemeinen enthalten die Formulierungen von 0,001 bis 98 Gew.-%, vorzugsweise 0,01 bis 95 Gew.-%, Wirkstoff. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile des Wirkstoffs werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
II. 20 Gewichtsteile der Verbindung Nr. I.01 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
III. 20 Gewichtsteile des Wirkstoffs werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. I.02 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser enthält man eine Spritzbrühe, die 0,1 Gew.% des Wirkstoffs enthält.
V. 3 Gewichtsteile des Wirkstoffs Nr. I.03 werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile des Wirkstoffs werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung Nr. I.04 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiiles Emulsionskonzentrat.
VIII. 1 Gewichtsteil der Verbindung Nr. I.05 wird in einer Mischung gelöst, die aus 80 Gewichtsteilen Cyclohexanon und 20 Gewichtsteilen Emulphor EL¹) besteht. Man erhält ein stabiles Emulsionskonzentrat.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

Zur Verbreiterung des Wirkungsspektrums und zur Erzielung synergistischer Effekte können die 3-(4-Cyanophenyl)uracile I mit zahlreichen Vertretern anderer herbizider oder wachstumsregulierender Wirkstoffgruppen gemischt und gemeinsam auagebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

### 1) ethoxyliertes Ricinusöl (caster-oil)

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### Beispiel 1

### Stufe 1

### 3-[4-Cyano-3-methoxy-phenyl]-6-trifluormethyl-1,2,3,4-tetrahydropyrimidin-2,4-dion

Zu einer Lösung von 3-[4-Cyano-3-nitro-phenyl]-6-trifluormethyl-1,2,3,4-tetrahydropyrimidin-2,4-dion (2,4 g) in 50 ml wasserfreiem Methanol wurde Natriummethanolatlösung (2,8 g einer 30 prozentigen Lösung in Methanol) gegeben. Dann erhitzte man das Reaktionsgemisch 5 Std. auf Rückflußtemperatur. Nach dem Abkühlen wurde erst Wasser (50ml) und dann bis zu einem pH-Wert von 3 -4 10 %ige wässrige Salzsäure zugegeben. Anschließend wurde der gebildete Niederschlag abgetrennt, mit Wasser und Petrolether gewaschen und getrocknet. Ausbeute: 1,1 g; Smp.: >230 °C.

### Stufe 2

### 1-Amino-3-[4-cyano-3-methoxy-phenyl]-6-trifluormethyl-1,2,3,4-tetrahydropyrimidin-2,4-dion (Verb.1.01)

Zu einer Lösung von 3-[4-Cyano-3-methoxy-phenyl]-6-trifluormethyl-1,2,3,4-tetrahydropyrimidin-2,4-dion (1,1g) in 15 ml Ethylacetat wurden Kaliumcarbonat (1,0 g) und 2,4-Dinitrophenoxyamin (0,8 g) gegeben. Anschließend rührte man 15 Std. bei 55-60 °C, wonach der gebildete Feststoffanteil abgetrennt und mit je 30 ml Ethylacetat und Diisopropylether gewaschen wurde. Die vereinigten Filtrate wurden zweimal mit je 25 ml Wasser gewaschen, über Natriumsulfat getrocknet und dann eingeengt. Nach Kristallisation mit 10 ml Diisopropylether erhielt man 0,6 g Wertprodukt. Smp.: >230°C.

### Beispiel 3

### 1-Amino-3-[4-cyano-3-hydroxy-phenyl]-6-trifluormethyl-1,2,3,4-tetrahydropyrimidin-2,4-dion (Verb.1.04)

1-Amino-3-[4-cyano-3-methoxy-phenyl]-6-trifluormethyl-1,2,3,4-tetrahydropyrimidin-2,4-dion (2,0 g) und Pyridiniumhydrochlorid (2,1 g) wurden 2 Std. bei 200-210°C gerührt. Nach dem Abkühlen löste man das Reaktionsgemisch in 100 ml n-Butanol, wonach die Lösung dreimal mit je 30 ml Wasser gewaschen wurde. Die organische Phase wurde über Natriumsulfat getrocknet und anschließend vom Lösungsmittel befreit. Nach Kristallisation mit 10 ml Diisopropylether und chromatographischer Reinigung des Rohproduktes (Laufmittel: Dichlormethan/Ethylacetat = 9:1 bis 1:1) erhielt man 0,4 g Wertprodukt. Smp.: > 230°C.

### Beispiel 4

### 3-[3-Allyloxy-4-cyano-phenyl]-1-methyl-6-trifluormethyl-1,2,3,4-tetrahydropyrimidin-2,4-dion (Verb. 1.05)

Zu einer Lösung von 3-[3-Allyloxy-4-cyano-phenyl]-1-methyl-6-trifluormethyl-1,2,3,4-tetrahydropyrimidin-2,4-dion (10,1 g) in 130 ml Dimethylformamid wurden Kaliumcarbonat (4,6 g) und Methyliodid (2,1 ml, gelöst in 20 ml Dimethylformamid) gegeben. Nach 20 stündigem Rühren bei Raumtemperatur versetzte man die Reaktionsmischung mit 150 ml Wasser, wonach der entstandene Niederschlag abgetrennt, mit Wasser und Petrolether gewaschen und getrocknet wurde. Ausbeute: 2,5 g; Smp.: 158-160°C.

In der folgenden Tabelle 2 sind neben den vorstehend genannten noch weitere 3-(4-Cyanophenyl)uracile I aufgeführt, die in analoger Weise hergestellt wurden oder herstellbar sind:

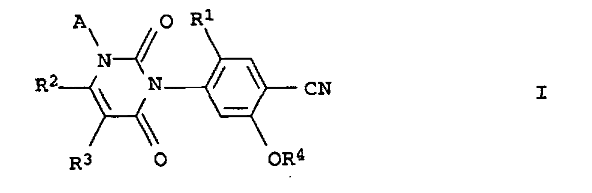

| Nr. | A | R¹ | R² | R³ | R⁴ | Smp. [°C] |
|---|---|---|---|---|---|---|
| 1.01 | NH₂ | H | CF₃ | H | CH₃ | >230 |
| 1.02 | NH₂ | H | CF₃ | H | CH₂CH=CH₂ | 177-179 |
| 1.03 | CH₃ | H | CF₃ | H | CH₃ | >230 |
| 1.04 | NH₂ | H | CF₃ | H | H | >230 |
| 1.05 | CH₃ | H | CF₃ | H | CH₂CH=CH₂ | 158-160 |
| 1.06 | NH₂ | H | CF₃ | H | CH(CH₃)₂ | 185-187 |

### Herstellung der Ausgangsverbindungen:

### Beispiel 5

### 3-[4-Cyano-3-nitro-phenyl]-6-trifluormethyl-1,2,3,4-tetrahydropyrimidin-2,4-dion

Zu einer Lösung von 3-[4-Fluor-3-nitro-phenyl]-6-trifluormethyl-1,2,3,4-tetrahydropyrimidin-2,4-dion (31,9 g) in 250 ml wasserfreiem N,N-Dimethylformamid wurden Kaliumcarbonat (16,6 g) und Kaliumcyanid (7,8 g) gegeben. Anschließend rührte man das Reaktionsgemisch insgesamt 45 Std. bei 75-80°C, wobei, da die Reaktion nicht vollständig abgelaufen war, noch zweimal Kaliumcyanaid (zusammen 4,6 g) zugegeben wurde. Zur Aufarbeitung gab man nach dem Abkühlen Wasser (250 ml) in das Reaktionsgemisch. Durch Zugabe von 60 ml 1 N Salzsäure wurde anschließend ein pH-Wert von 2-3 eingestellt. Nachdem zum Austreiben der freigestellten Blausäure 4 Std. Stickstoff durch die Suspension geblasen worden war, wurde der gebildete Niederschlag abgetrennt, mit Wasser und Petrolether gewaschen und getrocknet. Ausbeute: 17,0 g; Smp.: 135°C.

### Beispiel 6

### 3-[4-Cyano-2-fluor-5-nitro-phenyl]-1-methyl-6-trifluormethyl-1,2,3,4-tetrahydropyrimidin-2,4-dion

Zu einer Lösung von 3-[2,4-Difluor-5-nitro-phenyl]-1-methyl-6-trifluormethyl-1,2,3,4-tetrahydropyrimidin-2,4-dion (2,5 g) in 50 ml wasserfreiem Dimethylsulfoxid wurde Kaliumcyanid (0,5 g) gegeben. Anschließend rührte man das Reaktionsgemisch 10 Std. bei Raumtemperatur, wobei nach 5 Std. nochmals Kaliumcyanid (0,16 g) zugegeben wurde. Zur Aufarbeitung entfernte man das Lösungsmittel bei 80°C im Hochvakuum weitgehend. Der Rückstand wurde in 150 ml Wasser aufgenommen, dreimal mit je 30 ml Wasser gewaschen, über Natriumsulfat getrocknet und eingeengt. Nach Chromatographie an Kieselgel (Dichlormethan als Laufmittel) und Kristallisation mit Petrolether erhielt man 1,2 g Wertprodukt; Smp.: 155-157°C.

### Anwendungsbeispiele (herbizide Wirksamkeit)

Die herbizide Wirkung der 3-(4-Cyanophenyl)uracile I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,0156 oder 0,0078 kg/ha a.S. (aktive Substanz).

Die Pflanzen wurden artenspezifisch bei Temperaturen von 10 bis 25 °C bzw. 20 bis 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Galium aparine | Klettenlabkraut | catchweed bedstraw |
| Ipomoea subspecies | Prunkwindearten | morningglory |
| Solanum nigrum | Schwarzer Nachtschatten | black nightshade |

Bei einer Aufwandmenge von 0,0156 oder 0,0078 kg/ha a.S. zeigte die Verbindung Nr. I.01 im Nachauflaufverfahren eine sehr gute Wirkung gegen die o.g. Pflanzen.

### Anwendungsbeispiele (desikkative/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag/Nachttemperatur = 27/20°C).

Die jungen Baumwollpflanzen wurden tropfnaß mit wässrigen Aufbereitungen der Wirkstoffe (unter Zusatz von 0,15 Gew.-% des Fettalkoholalkoxylats Plurafac LF 700, bezogen auf die Spritzbrühe) blattbehandelt. Die ausgebrachte Wassermenge betrug umgerechnet 1000 1/ha. Nach 13 Tagen wurde die Anzahl der abgeworfenen Blätter und der Grad der Entblätterung in % bestimmt.

Bei den unbehandelten Kontrollpflanzen trat kein Blattfall auf.

## Patentansprüche

1. 3-(4-Cyanophenyl)uracile der allgemeinen Formel I 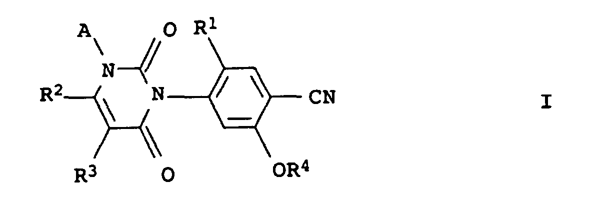 in der die Variablen folgende Bedeutungen haben:
R¹ Wasserstoff oder Fluor;
R² C₁-C₄-Halogenalkyl;
R³ Wasserstoff oder Halogen;
R⁴ Wasserstoff, C₁-C₄-Halogenalkyl, C₁-C₄-Alkyl, C₃-C₆-Cycloalkyl, C₃-C₄-Alkenyl, C₃-C₄-Alkinyl, (C₁-C₄-Alkyl)-carbonyl, C₁-C₄-Cyanoalkyl, C₁-C₄-Alkoxy-C₁-C₄-alkyl, CH₂-CO-XR⁵ oder CH(CH₃)-CO-XR⁵;
X eine chemische Bindung, Sauerstoff oder -N(R⁶)-;
R⁵ Wasserstoff, C₁-C₆-Alkyl, C₃-C₈-Cycloalkyl, C₃-C₆-Alkenyl, C₃-C₆-Alkinyl, C₁-C₆-Alkoxy-C₁-C₆-alkyl oder (C₁-C₆-Alkoxy)carbonyl-C₁-C₆-alkyl;
R⁶ Wasserstoff, C₁-C₆-Alkyl oder C₁-C₆-Alkoxy;
A Amino oder Methyl.

2. 3-(4-Cyanophenyl)uracile der Formel I nach Anspruch 1, wobei die Variablen folgende Bedeutung haben:
R³ Wasserstoff, Chlor oder Brom;
R⁵ Wasserstoff, C₁-C₆-Alkyl, C₃-C₈-Cycloalkyl, C₁-C₆-Alkoxy-C₁-C₆-alkyl oder (C₁-C₆-Alkoxy)carbonyl-C₁-C₆-alkyl;

3. 3-(4-Cyanophenyl)uracile der Formel I nach Anspruch 1, wobei A für Amino steht.

4. 3-(4-Cyanophenyl)uracile der Formel I nach Anspruch 1, wobei A für Wasserstoff oder Methyl steht.

5. 3-(4-Cyanophenyl)uracile der Formel I nach Anspruch 1, wobei R² für Trifluormethyl oder Chlordifluormethyl steht.

6. 3-(4-Cyanophenyl)uracile der Formel I nach Anspruch 1, wobei R⁴ für C₁-C₄-Alkyl, C₁-C₄-Cyanoalkyl oder C₃-C₄-Alkinyl steht.

7. Enamin-Ester der Formel III 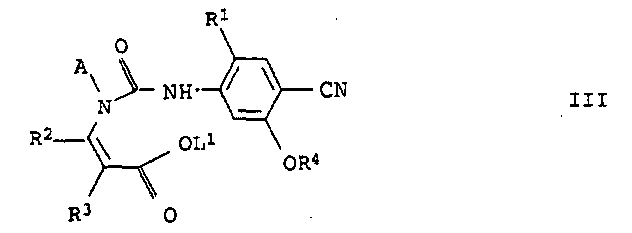 in der L¹ für C₁-C₆-Alkyl oder Phenyl steht und die Substituenten A und R¹ bis R⁴ die in Anspruch 1 angegebenen Bedeutungen haben.

8. Enamin-Carboxylate der Formel IV 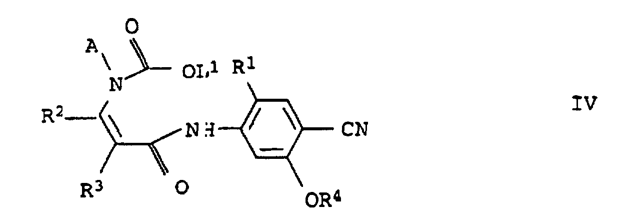 in der L¹ für C₁-C₆-Alkyl oder Phenyl steht und die Substituenten A und R¹ bis R⁴ die in Anspruch 1 angegebenen Bedeutungen haben.

9. Verwendung der 3-(4-Cyanophenyl)uracile der Formel I und der landwirtschaftlich brauchbaren Salze von I, gemäß Anspruch 1, als Herbizide oder zur Desikkation und/oder Defoliation von Pflanzen.

10. Herbizides Mittel, enthaltend eine herbizid wirksame Menge mindestens eines 3-(4-Cyanophenyl)uracils der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

11. Mittel zur Desikkation und/oder Defoliation von Pflanzen, enthaltend eine desikkant und/oder defoliant wirksame Menge mindestens eines 3-(4-Cyanophenyl)uracils der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

12. Verfahren zur Herstellung von herbizid wirksamen Mitteln, **dadurch gekennzeichnet, daß** man eine herbizid wirksame Menge mindestens eines 3-(4-Cyanophenyl)uracils der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

13. Verfahren zur Herstellung von desikkant und/oder defoliant wirksamen Mitteln, **dadurch gekennzeichnet, daß** man eine desikkant und/oder defoliant wirksame Menge mindestens eines 3-(4-Cyanophenyl)uracils der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

14. Verfahren zur Bekämpfung von unerwünschtem Pflanzenwuchs, **dadurch gekennzeichnet, daß** man eine herbizid wirksame Menge mindestens eines 3-(4-Cyanophenyl)uracils der Formel I oder eines landwirtschaftlich brauchbaren Salzes von I, gemäß Anspruch 1, auf Pflanzen, deren Lebensraum oder auf Saatgut einwirken läßt.

15. Verfahren zur Desikkation und/oder Defoliation von Pflanzen, **dadurch gekennzeichnet, daß** man eine desiccant und/oder defoliant wirksame Menge mindestens eines 3-(4-Cyanophenyl)uracils der Formel I oder eines landwirtschaftlich brauchbaren Salzes von I, gemäß Anspruch 1, auf Pflanzen einwirken läßt.

16. Verfahren zur Herstellung von 3-(4-Cyanophenyl)uracilen der Formel I gemäß Anspruch 1, **dadurch gekennzeichnet, daß** man entweder
a) einen Enamin-Ester der Formel III 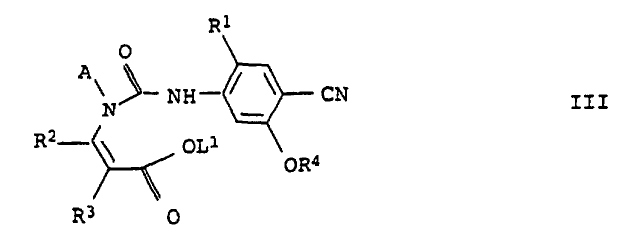 oder ein Enamin-Carboxylat der Formel IV 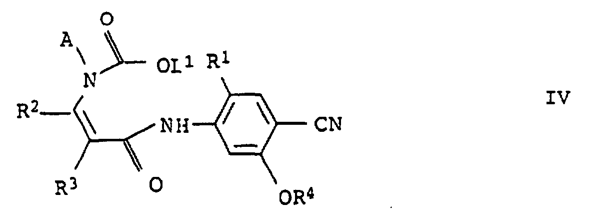 cyclisiert;
b) ein 3-(4-Cyanophenyl)uracil der Formel I, bei dem A Wasserstoff bedeutet, methyliert oder aminiert;
c) das Halogenid an Verbindungen der Formel V 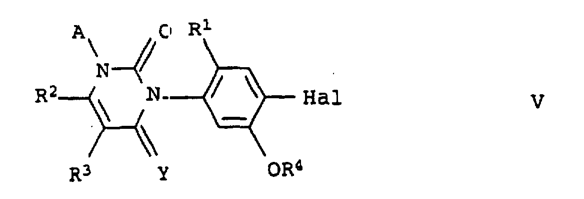 durch Cyanid substituiert;
d) ein 3-(4-Cyanophenyl)uracil der Formel I, bei dem R³ Wasserstoff bedeutet, halogeniert;
e) ein 3-(4-Cyanophenyl)uracil der Formel I, bei dem R⁴ Wasserstoff bedeutet, alkyliert oder acyliert;
f) ein 3-(4-Cyanophenyl)uracil der Formel I, bei dem R⁴ nicht für Wasserstoff steht, einer Ether- oder Esterspaltung unterwirft;
g) ein 3-(4-Halogenphenyl)uracil der Formel VIII 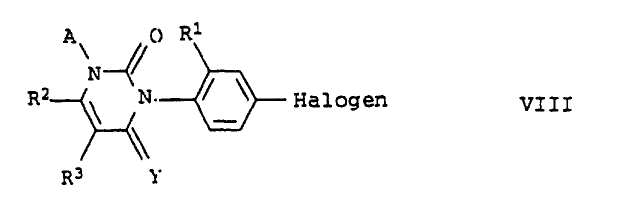 nitriert, anschließend das Halogenatom durch Cyano und die Nitro-Gruppe durch ^{⊖}OR⁴ substituiert;
h) oder eine Phenylverbindung der Formel X 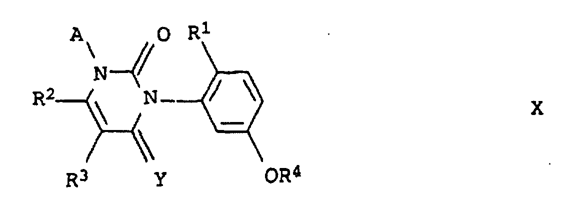 nitriert, die Nitrogruppe anschließend zur Aminogruppe reduziert und diese dann nach der Methode von Sandmeyer in die Cyanogruppe überführt.

## Claims

1. A 3-(4-cyanophenyl)uracil of the general formula I 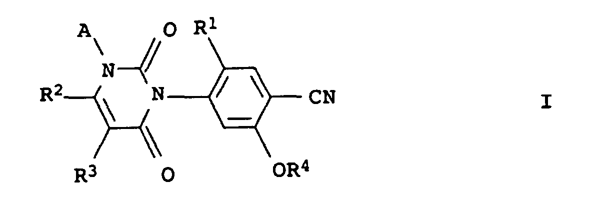 where the variables have the following meanings:
R¹ is hydrogen or fluorine;
R² is C₁-C₄-haloalkyl;
R³ is hydrogen or halogen;
R⁴ is hydrogen, C₁-C₄-haloalkyl, C₁-C₄-alkyl, C₃-C₆-cycloalkyl, C₃-C₄-alkenyl, C₃-C₄-alkynyl, (C₁-C₄-alkyl)carbonyl, C₁-C₄-cyanoalkyl, C₁-C₄-alkoxy-C₁-C₄-alkyl, CH₂-CO-XR⁵ or CH(CH₃)-CO-XR⁵;
X is a chemical bond, oxygen or -N(R⁶)-;
R⁵ is hydrogen, C₁-C₆-alkyl, C₃-C₈-cycloalkyl, C₃-C₆-alkenyl, C₃-C₆-alkynyl, C₁-C₆-alkoxy-C₁-C₆-alkyl or (C₁-C₆-alkoxy)carbonyl-C₁-C₆-alkyl;
R⁶ is hydrogen, C₁-C₆-alkyl or C₁-C₆-alkoxy.
A is amino or methyl.

2. A 3-(4-cyanophenyl)uracil of the formula I as claimed in claim 1, where the variables have the following meaning:
R³ is hydrogen, chlorine or bromine;
R⁵ is hydrogen, C₁-C₆-alkyl, C₃-C₈-cycloalkyl, C₁-C₆-alkoxy-C₁-C₆-alkyl or (C₁-C₆-alkoxy)carbonyl-C₁-C₆-alkyl.

3. A 3-(4-cyanophenyl)uracil of the formula I as claimed in claim 1 where A is amino.

4. A 3-(4-cyanophenyl)uracil of the formula I as claimed in claim 1 where A is hydrogen or methyl.

5. A 3-(4-cyanophenyl)uracil of the formula I as claimed in claim 1, where R² is trifluoromethyl or chlorodifluoromethyl.

6. A 3-(4-cyanophenyl)uracil of the formula I as claimed in claim 1, where R⁴ is C₁-C₄-alkyl, C₁-C₄-cyanoalkyl or C₃-C₄-alkynyl.

7. An enamine ester of the formula III 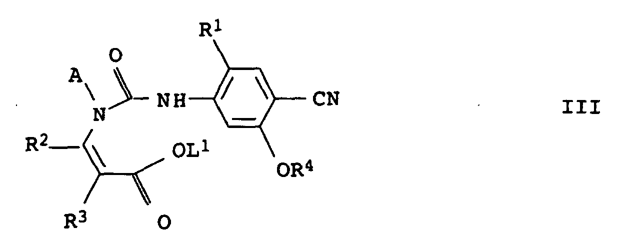 where L¹ is C₁-C₆-alkyl or phenyl and the substituents A and R¹ to R⁴ have the meanings given in claim 1.

8. An enamine carboxylate of the formula IV 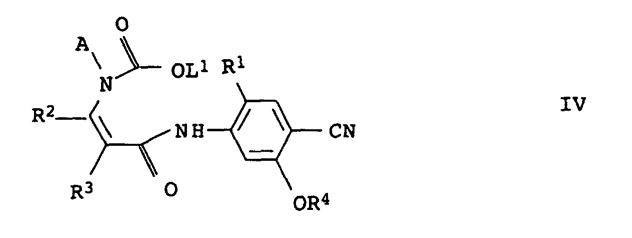 where L¹ is C₁-C₆-alkyl or phenyl and the substituents A and R¹ to R⁴ have the meanings given in claim 1.

9. The use of the 3-(4-cyanophenyl)uracils of the formula I and of the agriculturally useful salts of I as claimed in claim 1 as herbicides or for the desiccation and/or defoliation of plants.

10. Herbicidal composition comprising a herbicidally effective amount of at least one 3-(4-cyanophenyl)uracil of the formula I or of an agriculturally useful salt of I as claimed in claim 1 and at least one inert liquid and/or solid carrier and, if desired, at least one surfactant.

11. A composition for the desiccation and/or defoliation of plants comprising such an amount of at least one 3-(4-cyanophenyl)uracil of the formula I or of an agriculturally useful salt of I as claimed in claim 1 that it acts as a desiccant and/or defoliant, and at least one inert liquid and/or solid carrier and, if desired, at least one surfactant.

12. A process for the preparation of herbicidally active compositions, which comprises mixing a herbicidally active amount of at least one 3-(4-cyanophenyl)uracil of the formula I or of an agriculturally useful salt of I as claimed in claim 1 and at least one inert liquid and/or solid carrier, and, if desired, at least one surfactant.

13. A process for the preparation of compositions which act as desiccants and/or defoliants, which comprises mixing such an amount of at least one 3-(4-cyanophenyl)uracil of the formula I or of an agriculturally useful salt of I as claimed in claim 1 that it acts as a desiccant and/or defoliant, and at least one inert liquid and/or solid carrier, and, if desired, at least one surfactant.

14. A method of controlling undesirable vegetation, which comprises allowing a herbicidally active amount of at least one 3-(4-cyanophenyl)uracil of the formula I or of an agriculturally useful salt of I as claimed in claim 1 to act on plants, their environment or on seed.

15. A method of desiccating and/or defoliating plants, which comprises applying to plants such an amount of at least one 3-(4-cyanophenyl)uracil of the formula I or of an agriculturally useful salt of I as claimed in claim 1 that it acts as a desiccant and/or defoliant.

16. A process for the preparation of 3-(4-cyanophenyl)uracils of the formula I as claimed in claim 1, which comprises either
a) cyclizing an enamine ester of the formula III 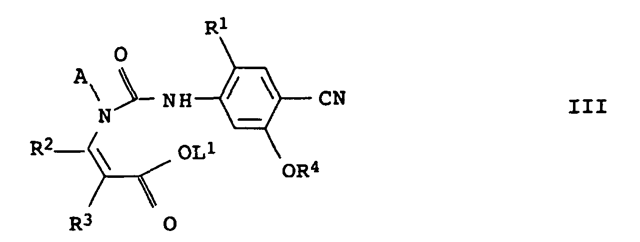 or an enamine carboxylate of the formula IV 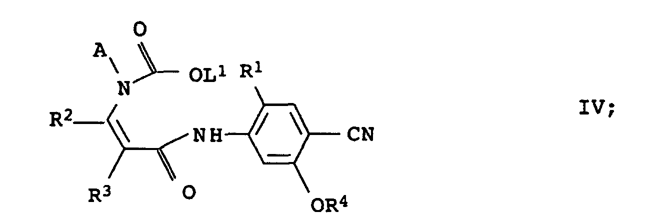
b) methylating or aminating a 3-(4-cyanophenyl)uracil of the formula I where A is hydrogen;
c) substituting the halide in compounds of the formula V 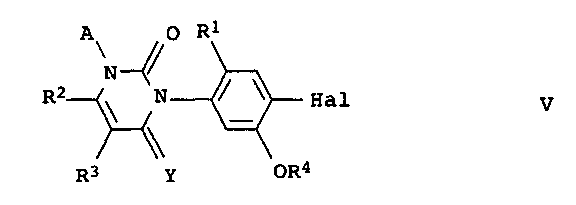 by cyanide;
d) halogenating a 3-(4-cyanophenyl)uracil of the formula I where R³ is hydrogen;
e) alkylating or acylating a 3-(4-cyanophenyl)uracil of the formula I where R⁴ is hydrogen;
f) subjecting a 3-(4-cyanophenyl)uracil of the formula I where R⁴ is other than hydrogen to ether or ester cleavage;
g) nitrating a 3-(4-halophenyl)uracil of the formula VIII 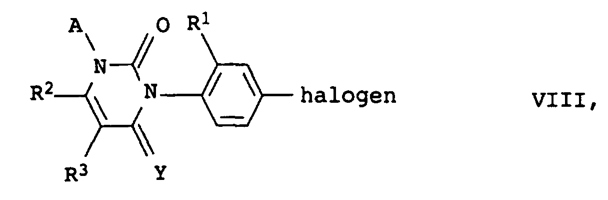 subsequently substituting the halogen atom by cyano and the nitro group by ^{⊖}OR⁴;
h) or nitrating a phenyl compound of the formula X 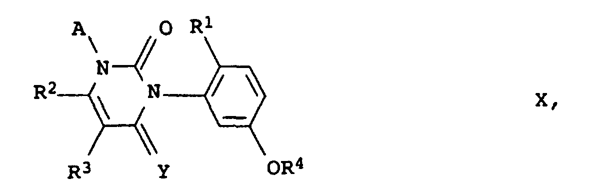 subsequently reducing the nitro group to the amino group and then converting the latter into the cyano group by the method of Sandmeyer.

## Revendications

1. 3-(4-cyanophényl)uraciles de formule générale I 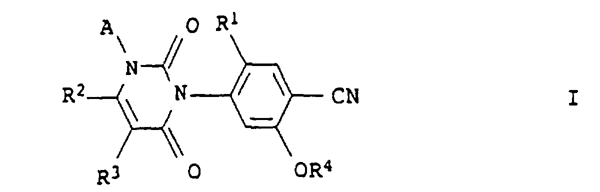 dans laquelle les symboles ont les significations suivantes:
R¹ : l'hydrogène ou le fluor ;
R² : un groupe halogénoalkyle en C₁-C₄;
R³ : l'hydrogène ou un halogène ;
R⁴ : l'hydrogène, un groupe halogénoalkyle en C₁-C₄, alkyle en C₁-C₄, cycloalkyle en C₃-C₆, alcényle en C₃-C₄, alcynyle en C₃-C₄, (alkyle en C₁-C₄)carbonyle, cyanoalkyle en C₁-C₄, (alcoxy en C₁-C₄)alkyle en C₁-C₄, CH₂-CO-XR⁵ ou CH(CH₃)-CO-XR⁵ ;
X: une liaison chimique, l'oxygène ou -N(R⁶)- ;
R⁵ : l'hydrogène, un groupe alkyle en C₁-C₆, cycloalkyle en C₃-C₈, alcényle en C₃-C₆, alcynyle en C₃-C₆, (alcoxy en C₁-C₆)alkyle en C₁-C₆ ou (alcoxy en C₁-C₆)carbonylalkyle en C₁-C₆ ;
R⁶ : l'hydrogène, un groupe alkyle en C₁-C₆ ou alcoxy en C₁-C₆ ;
A : un groupe amino ou méthyle.

2. 3-(4-cyanophényl)uraciles de formule I de la revendication 1 dans laquelle les symboles ont les significations suivantes:
R³ : l'hydrogène, le chlore ou le brome;
R⁵ : l'hydrogène, un groupe alkyle en C₁-C₆, cycloalkyle en C₃-C₈, (alcoxy en C₁-C₆)-alkyle en C₁-C₆ ou (alcoxy en C₁-C₆)carbonyl-alkyle en C₁-C₆.

3. 3-(4-cyanophényl)uraciles de formule I de la revendication 1, dans laquelle A représente un groupe amino.

4. 3-(4-cyanophényl)uraciles de formule I de la revendication 1, dans laquelle A représente l'hydrogène ou un groupe méthyle.

5. 3-(4-cyanophényl)uraciles de formule I de la revendication 1, dans laquelle R² représente un groupe trifluorométhyle ou chlorodifluorométhyle.

6. 3-(4-cyanophényl)uraciles de formule I de la revendication 1, dans laquelle R⁴ représente un groupe alkyle en C₁-C₄, cyano-alkyle en C₁-C₄ ou alcynyle en C₃-C₄.

7. Enamine-esters de formule III 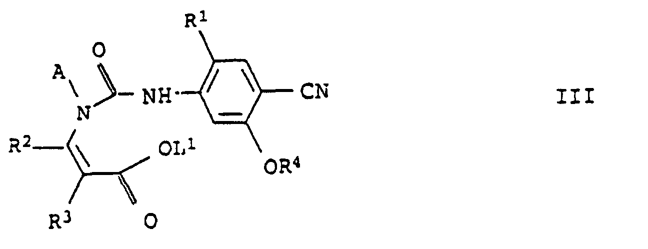 dans laquelle L¹ représente un groupe alkyle en C₁-C₆ ou phényle et les symboles A et R¹ à R⁴ ont les significations indiquées dans la revendication 1.

8. Enamine-carboxylates de formule IV 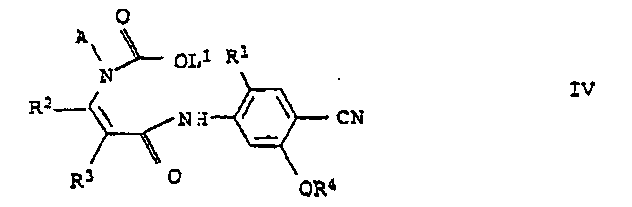 dans laquelle L¹ représente un groupe alkyle en C₁-C₆ ou phényle et les symboles A et R¹ à R⁴ ont les significations indiquées dans la revendication 1.

9. Utilisation des 3-(4-cyanophényl)uraciles de formule I et de leurs sels aptes aux application agricoles selon la revendication 1 en tant qu'herbicides ou pour la dessiccation et/ou la défoliation de végétaux.

10. Produit herbicide contenant une quantité herbicide efficace d'au moins un 3-(4-cyanophényl)uracile de formule I ou de l'un de ses sels apte aux applications agricoles selon la revendication 1 et au moins un véhicule liquide et/ou solide inerte, avec le cas échéant au moins une substance tensio-active.

11. Produit pour la dessiccation et/ou la défoliation de végétaux, contenant une quantité dessiccante et/ou défoliante efficace d'au moins un 3-(4-cyanophényl)uracile de formule I ou de l'un de ses sels aptes aux applications agricoles selon la revendication 1 et au moins un véhicule liquide et/ou solide inerte avec le cas échéant au moins une substance tensio-active.

12. Procédé pour la préparation de produits à activité herbicide **caractérisé par le fait que** l'on mélange une quantité herbicide efficace d'au moins un 3-(4-cyanophényl)uracile de formule I ou d'un de ses sels aptes aux applications agricoles selon la revendication 1 et au moins un véhicule liquide et/ou solide inerte ainsi que, si on le désire, au moins une substance tensio-active.

13. Procédé pour la préparation de produits à activité dessiccante et/ou défoliante **caractérisé par le fait que** l'on mélange une quantité dessiccante et/ou défoliante efficace d'au moins un 3-(4-cyanophényl)uracile de formule I ou de l'un de ses sels aptes aux applications agricoles selon la revendication 1 et au moins un véhicule liquide et/ou solide inerte avec, si on le désire, au moins une substance tensio-active.

14. Procédé pour combattre les croissances de végétaux indésirables, **caractérisé par le fait que** l'on fait agir une quantité herbicide efficace d'au moins un 3-(4-cyanophényl)uracile de formule I ou de l'un de ses sels selon la revendication 1 sur les végétaux, leur habitat ou sur les semences.

15. Procédé pour la dessiccation et/ou la défoliation de végétaux, **caractérisé par le fait que** l'on fait agir une quantité dessiccante et/ou défoliante efficace d'au moins un 3-(4-cyanophényl)uracile de formule I ou de l'un de ses sels aptes aux applications agricoles selon la revendication 1 sur les végétaux.

16. Procédé pour la préparation des 3-(4-cyanophényl)uraciles de formule I de la revendication 1 **caractérisé par le fait que**
a) on cyclise un énamine-ester de formule III 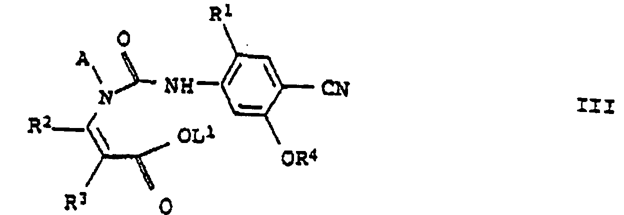 ou un énamine-carboxylate de formule IV 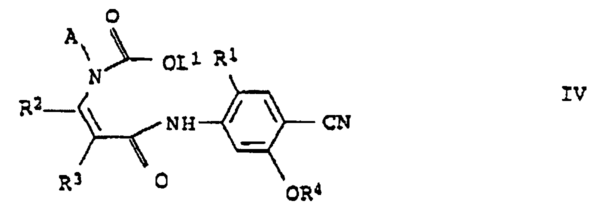 ou bien
b) on méthyle ou on amine un 3-(4-cyanophényl)uracile de formule I dans laquelle A représente l'hydrogène,
ou bien
c) on remplace l'halogène de composés de formule V 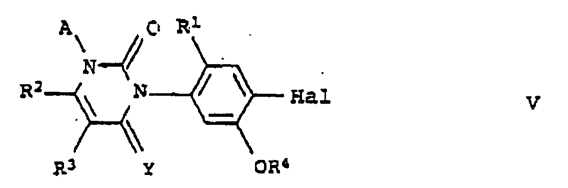 par un cyanure ;
ou bien
d) on halogène un 3-(4-cyanophényl)uracile de formule I dans laquelle R³ représente l'hydrogène;
ou bien
e) on alkyle ou on acyle un 3-(4-cyanophényl)uracile de formule I dans laquelle R⁴ représente l'hydrogène;
ou bien
f) on soumet un 3-(4-cyanophényl)uracile de formule I dans laquelle R⁴ a une signification autre que l'hydrogène, à scission d'un groupe éther ou ester;
ou bien
g) on nitre un 3-(4-halogénophényl)uracile de formule VIII 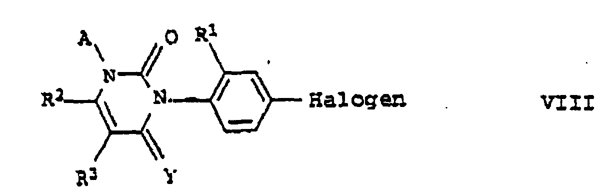 puis on remplace l'atome d'halogène par un groupe cyano et le groupe nitro par un groupe ^{⊖}OR⁴;
ou bien
h) on nitre un dérivé phénylique de formule X 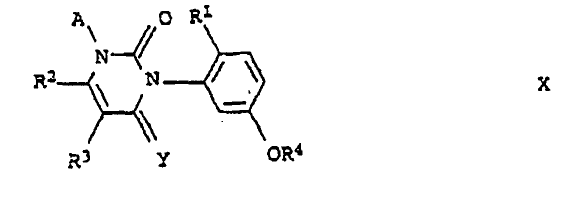 on réduit ensuite le groupe nitro en groupe amino puis on convertit ce dernier en groupe cyano par le procédé de Sandmeyer.
